# EUROPÄISCHE PATENTANMELDUNG

(11) **EP 3 220 141 A1**
(43) Veröffentlichungstag der Anmeldung: **20.09.2017**
(21) Anmeldenummer: 17000356.0
(22) Anmeldetag: 07.03.2017
(51) Int. Cl.: G01N 33/00

(54) **VERFAHREN ZUR ÜBERPRÜFUNG EINES GASSENSORS UND GASMESSVORRICHTUNG, SOWIE GASWARNANLAGE MIT EINER PRÜFVORRICHTUNG ZUR ÜBERPRÜFUNG EINES GASSENSORS**

(30) Priorität: 18.03.2016 DE 102016003283
(71) Anmelder: Dräger Safety AG & Co. KGaA, 23560 Lübeck (DE)
(72) Erfinder: Diekmann, Wilfried, 19217 Utecht (DE)
(74) Vertreter: Heinemeyer, Karsten

(57) **Zusammenfassung**

Ein Verfahren zur Überprüfung eines Gassensors (30) und eine Gasmessvorrichtung mit einer Prüfvorrichtung zur Überprüfung des Gassensors (30) ermöglichen eine verbesserte Analyse und Bewertung von Zuständen von

Gassensoren (30). Durch eine Überprüfung eines Gaszutrittselements (8) mittels einer Überwachung von Messsignalen (35, 38) in einem Zeitverlauf (400) und ein Vergleich mit Schwellwerten (350, 351) zu vorbestimmten Zeitpunkten (403, 404), (403", 404") in Verbindung mit einer Dosierung (91) einer Prüfstoffmenge (5, 6) ermöglicht es, zu überprüfen, ob ein Gaszutritt (7) zu dem Gassensor (30, 309) möglich und gegeben ist.

## Beschreibung

Die vorliegende Erfindung betrifft ein Verfahren zur Überprüfung eines Gassensors sowie eine Prüfvorrichtung zur Überprüfung des Gassensors in einer Gasmessvorrichtung.
Gasmessvorrichtungen werden zur technischen Gasmessung eingesetzt und schützen Personen, welche sich in Industriearealen oder in Gebäuden aufhalten, in denen mögliche gesundheitsgefährdende Gase, sei es Prozessgase oder Abgase vorhanden sein können, vor Gefahren für Gesundheit und Leben.

Durch Gasmessvorrichtungen werden im Umfeld der Industrie, beispielsweise der chemischen oder petrochemischen Industrie technische Gase dahingehend überwacht, ob von diesen Gasen eine Gefahr aufgrund explosiver oder giftiger Eigenschaften ausgeht. Zum Einsatz kommen dabei in vielen Fällen stationär eingesetzte Gasmessgeräte oder Gasmessvorrichtungen. Als Sensoren werden in solchen Gasmessvorrichtungen üblicherweise infrarot-optisch messende Sensoren, elektrochemische Sensoren, katalytische Sensoren oder Halbleitergassensoren eingesetzt.
Wichtig für die Verlässlichkeit der Gasmessvorrichtungen, sowie der dadurch erzeugten Alarme, Störungsmeldungen und Warnungen ist es, dass die Gasmessvorrichtungen im Betrieb voll funktionsfähig sind und, dass Fehlfunktionen dabei sicher erkannt werden können.

In der US 4 338 28 A wird ein Dünnschicht-Hableitergassensor beschrieben. Der Dünnschicht-Halbleitergassensor weist ein integriertes Heizelement auf. Es handelt sich dabei um einen Metalloxid-Halbleiter, bei dem der elektrische Widerstand der Metalloxid-Halbleiterschicht in Abhängigkeit von der Konzentration des zu detektierenden Gases abhängig ist. Diese Widerstandsänderung ist als Maß für die Anwesenheit des zu detektierenden Gases messtechnisch durch eine geeignete, dem Dünnschicht-Hableitergassensor zugeordnete Auswerteelektronik erfassbar.

In der US 4 854 155 A wird eine katalytisch arbeitende Detektorschaltung für brennbare Gase beschrieben. Dabei sind die Detektoren für die brennbaren Gase als Sensorwiderstandselemente mit katalytischer Beschichtung ausgebildet. Detektoren für brennbare Gase werden dazu verwendet, um das Vorhandensein brennbarer Gase zu detektieren, wie sie beispielsweise in Bergwerken oder Industrieanlagen auftreten können.

In der US 5 565 075 A wird ein elektrochemischer Gassensor für die Erfassung von Stickstoff-Monoxid beschrieben. Der Sensor weist in einem mit einem Elektrolyten gefüllten Gehäuse eine Arbeitselektrode, eine Referenzelektrode und eine Gegenelektrode auf.
Ein solcher elektrochemischer Gassensor ist vorzugsweise einsetzbar in medizinischen Anwendungen, da er mit einer geringen Querempfindlichkeit gegen andere in dieser Umgebung üblicherweise verwendeten Gase ausgestattet ist.

In der US 2005/0247878 A1 ist ein Infrarot-Gassensor beschrieben. In einem Gehäuse sind zwei nebeneinander angeordnete infrarote Strahlungsdetektoren auf einer Seite des Gehäuses angeordnet, während auf der anderen Seite eine Strahlungsquelle angeordnet ist, welche infrarote Strahlung emittiert. Zu analysierendes Gas wird aus einer Messumgebung in den Strahlengang eingebracht.
Der Messeffekt basiert darauf, dass die Dämpfung des von der Strahlungsquelle abgestrahlten Lichtes abhängig von der Gasart im Strahlengang erfolgt. Einer der beiden Detektoren wird als ein Referenzdetektor betrieben, während der andere Detektor als ein Messdetektor betrieben wird. Aus dem Verhältnis der Signale des Messdetektors und des Referenzdetektors wird die Gaskonzentration des in den Strahlengang eingebrachten Gases bestimmt.

In vielen Anwendungssituationen werden die genannten Gassensoren zu stationären Gasmesseinrichtungen zusammengefasst oder weitergebildet. Stationäre Gasmesseinrichtungen sind oftmals und üblicherweise in Industrieanlagen als eine Vielzahl von Sensoreinheiten zur Gasmessung über eine Mehrzahl von Räumen oder größere Areale verteilt.

Die US 6 182 497 B1 beschreibt ein Gasmesssystem, das ausgebildet ist, eine Vielzahl von Sensoren an einer zentralen Auswerteeinheit anzuschließen. Der Anschluss der Sensoren kann dabei beispielsweise über einen universellen, seriellen Bus erfolgen.

Aus der US 7 406 854 B2 ist ein Gassensor mit einem Adapter bekannt. Der Adapter ist zu einem Anschluss einer Schlauchleitung ausgebildet. Über diese Schlauchleitung ist es möglich, Gase von einem entfernt liegenden Messort oder ein Messgas oder ein Kalibriergas zu einer Überprüfung einer Funktionsfähigkeit des Gassensors an den Gassensor heranzuführen. Diese Heranführung von Messgas oder Kalibriergas kann dabei beispielsweise mittels einer Förderpumpe erfolgen.

In der CH 624 488 A5 ist ein Verfahren zur Überprüfung von Gasanalysegeräten beschrieben. Mit Hilfe eines Testgaskonzentrationsstoßes wird da durch den Testgasstoß hervorgerufene Messsignal hinsichtlich Empfindlichkeit und Zeitverhalten ausgewertet. Zusätzlich kann mit Hilfe einer Wiederholung von Testgaskonzentrationsstößen das Regenerationsverhalten des Gasanalysegerätes untersucht werden.

In der GB 2 356 708 A ist ein Sensorsystem mit einem Sensor, beispielsweise zur Messung von Kohlenmonoxid bekannt, in welchem eine Öffnung zur Zuführung eines Testgases vorgesehen ist, um einen Test hinsichtlich der Funktionsweise des Sensors anhand der Signalantwort des Sensors vorzunehmen.

In der US 2013/019 233 A1 ist eine Art und Weise einer Sensorüberprüfung beschrieben, anhand von Sensorreaktionen oder Signalantworten in Bezug zu vorbestimmten Schwellenwerten einen Zustand des Sensors abzufragen und eine automatische Kalibrierung vorzunehmen.

Aus der WO 1999/17110 A1, der DE 10 2009 052 957 A1 wie auch aus der US 7 645 367 B2 sind Gasmesssysteme, bestehend aus einem Gassensor und einem Gasgenerator bekannt. Solcherlei Kombinationen von Gasgeneratoren und Gassensoren ermöglichen eine Überprüfung der Messeigenschaften der Gassensoren, insbesondere dahingehend, ob der Gassensor auf eine Beaufschlagung mit einer vorgegebenen Messgaskonzentration sensitiv reagiert.

Eine Vorrichtung zu einer Überprüfung eines Gassensors ist beispielsweise aus der DE 20 2006 020 536 U1 bekannt. Darin wird ein Gasgenerator beschrieben, der zu einer Erzeugung von Ethan geeignet ist. Der Gasgenerator ist zu einer Überprüfung des Gassensors vorgesehen und ausgebildet, eine gewisse Menge eines Test- oder Prüfgases an/in den Gassensor zu dosieren, wobei eine darauf basierende Änderung oder Reaktion des Ausgangssignals des Gassensors einen Hinweis auf die Funktionsfähigkeit des Gassensors darstellt.

Von Seiten der Amerikanischen Arbeitsschutzbehörde (OSHA) gibt es Empfehlungen hinsichtlich zu Funktionstests mit sogenannten "Bump-tests", wobei mittels geeigneter Adapter und einem geeigneten Testgas eine regelmäßige Überprüfung von Gassensoren durchzuführen ist.

Die US 7 406 854 B2 beschreibt einen Adapter zu einer Überprüfung oder zu einer Kalibrierung eines elektrochemischen Gassensors. Der Adapter ist vorzugsweise mit einem Klettverschluss am Gassensor anbringbar und nach erfolgter Überprüfung oder Kalibrierung wieder von diesem entfernbar.

Insbesondere für bereits bestehende Anlagen oder Installationen von Gasmesssystemen ist ein Bedürfnis vorhanden, die Funktionsfähigkeit der Gassensoren im Betrieb regelmäßig zu überprüfen. Insbesondere ist ein Bedürfnis vorhanden, die Überprüfung der Gassensoren vornehmen zu können, ohne, dass ein Ausbau oder eine Demontage der Gassensoren am jeweiligen Messort der Anlage erforderlich ist. Die Überprüfung von Gassensoren in bereits bestehenden Gasmesssystemen soll dabei ohne aufwändiges manuelles Zutun durch Wartungspersonal ermöglicht sein, wie es beispielsweise bei einer Verwendung des Adapters nach der US 7 406 854 B2 notwendig sein würde, da ansonsten für jeden Gassensor in einer größeren Industrieanlage vom Wartungspersonal eine Anbringung mit nachfolgender Sensorüberprüfung und anschließender Entfernung des Adapters mit Inbetriebnahme des Gassensors manuell erfolgen müsste. Weiterhin ist ein Bedürfnis vorhanden, festzustellen, dass Gassensoren, welche einen Gaskonzentrationsmesswert als Ausgangssignal liefern, welcher auf eine Abwesenheit von Schadgasen, also eine störungsfreie Situation schließen lässt, mit den sensorisch wirksamen Elementen auch in einem wirksamen Gasaustausch mit einer Messumgebung am Messort stehen. Es ist also ein Bedürfnis gegeben, Fehlinterpretationen, insbesondere von gleichbleibenden, unkritischen Gaskonzentrationsmesswerten oder anderen gleichbleibenden Ausgangssignalen des Gassensors zu vermeiden.

Unter Kenntnis des vorgenannten Standes der Technik ergibt sich als Aufgabe der vorliegenden Erfindung, ein Verfahren zu einer Überprüfung eines Gassensors bereitzustellen, welches es ermöglicht, festzustellen, ob ein ungehinderter Zutritt von Gas aus einer Messumgebung zu dem Gassensor möglich ist.

Eine weitere Aufgabe der vorliegenden Erfindung ist es, eine Gasmessvorrichtung mit einer Prüfvorrichtung anzugeben, welche eine Durchführung des Verfahrens zu einer Überprüfung eines Gassensors ermöglicht.

Eine weitere Aufgabe der vorliegenden Erfindung ist es, ein Gaswarnsystem anzugeben, welche eine Überprüfung eines ungehinderten Zutritts von Gas aus einer Messumgebung zu einem Gassensor ermöglicht.

Diese und weitere Aufgaben werden mit Hilfe der nebengeordneten Patentansprüche, insbesondere mit einem Verfahren zur Überprüfung eines Gassensors mit den Merkmalen des Patentanspruchs 1 gelöst.

Die Aufgabe wird weiterhin mit einer zur Durchführung des Verfahrens geeigneten Gasmessvorrichtung mit den Merkmalen des Patentanspruchs 15 gelöst.

Die Aufgabe wird weiterhin mit einem Gaswarnsystem mit den Merkmalen des Patentanspruchs 21 gelöst.

Dabei gelten Merkmale und Details, die im Zusammenhang mit dem erfindungsgemäßen Verfahren zur Überprüfung des Gassensors beschrieben sind, selbstverständlich auch im Zusammenhang und im Hinblick auf die Durchführung des Verfahrens geeignete Gasmessvorrichtung und das erfindungsgemäße Gaswarnsystem und jeweils umgekehrt, so dass bezüglich der Offenbarung zu den einzelnen Aspekten der Erfindung stets wechselseitig Bezug genommen wird, bzw. werden kann.

Vorteilhafte Ausführungsformen ergeben sich aus den Unteransprüchen und werden in der folgenden Beschreibung unter teilweise Bezugnahme der Figuren näher erläutert.

Grundlage der vorliegenden Erfindung ist, dass in einer Anordnung zur Gasmessung oder einer Gasmessvorrichtung an einem Gassensor angeordnete Einheit zur Prüfgasdosierung derart betrieben wird, um zu überwachen oder zu kontrollieren, ob ein ungehinderter Gaszutritt durch mindestens ein in der Anordnung zur Gasmessung oder Gasmessvorrichtung vorhandenes Gaszutrittselement in den Gassensor hinein gegeben ist und somit zu überprüfen, ob die Funktionsbereitschaft des Gassensors gegeben ist.

Zu Beginn werden einige der im Rahmen dieser Patentanmeldung verwendeten Begrifflichkeiten näher erläutert.

Unter einem Ausgangssignal oder einem Messsignal, wie auch einem Sensorsignal ist im Sinne der vorliegenden Erfindung ein Signal zu verstehen, welches vom Gassensor bereitgestellt wird und welches einen Gaskonzentrationswert repräsentiert. Das Ausgangssignal oder das Messsignal können dabei als ein Spannungssignal, z.B. in einem Spannungsbereich von 0V ... 10V, ein Stromsignal, z.B. als ein sogenanntes 4 mA...20 mA- Ausgangssignal, als digitale Datenworte mittels Datenleitungen und/oder verschiedener Schnittstellen und Protokolle, wie RS232, RS485, NMEA183, USB, CAN-Bus, LAN, WLAN, TCP/IP, Ethernet, Bluetooth vom Gassensor bereitgestellt werden.

Unter einem Steuersignal wird im Sinne der vorliegenden Erfindung ein einzelnes Steuersignal, ein Steuersignal als Teil einer Menge von Steuersignalen, wie auch eine Vielzahl oder eine Menge von Steuersignalen verstanden.

Unter einem Datensignal wird im Sinne der vorliegenden Erfindung ein einzelnes Datensignal, ein Datensignal als Teil einer Menge von Datensignalen, wie auch eine Vielzahl oder eine Menge von Datensignalen verstanden.

Unter einem Ausgabesignal wird im Sinne der vorliegenden Erfindung ein einzelnes Ausgabesignal, ein Ausgabesignal als Teil einer Menge von Ausgabesignalen, wie auch eine Vielzahl oder eine Menge von Ausgabesignalen verstanden. Eine Datenverbindung ist im Sinne der vorliegenden Erfindung eine Verbindung von mindestens zwei Teilnehmern mittels einer draht- gebundenen, drahtlosen, optischen Verbindung, welche zu einer Übermittlung von Ausgangssignalen, Steuersignalen, Datensignalen oder Ausgabesignalen geeignet ist. Dabei sind sowohl direkte physikalische Verbindungen (Kabelverbindungen, Funkverbindungen, Lichtleiterverbindungen), wie auch indirekte oder logische Verbindungen zur Übermittlung von Informationen, Steuersignalen, Datensignalen oder Ausgabesignalen mit physikalischen oder Datentechnischen Wandlungen oder Umwandlungen von Signalen, Spannungen, Strömen mit umfasst.

Im Sinne der vorliegenden Erfindung ist unter einem Messgas ein Gas oder ein Gasgemisch zu verstehen, das derart beschaffen ist, dass der mindestens eine Gassensor auf eine Änderung einer Gaskonzentration dieses Messgases empfindlich ist und auf Änderungen in der Gaskonzentration dieses Messgases mit Änderungen des Gaskonzentrationsmesswertes reagiert.

Im Sinne der vorliegenden Erfindung ist unter einem Prüfgas ein Gas oder ein Gasgemisch zu verstehen, das derart beschaffen ist, dass der mindestens eine Gassensor auf eine Änderung einer Gaskonzentration dieses Gases oder Gasgemisches empfindlich ist und auf Änderungen in der Gaskonzentration dieses Prüfgases mit Änderungen des Gaskonzentrationsmesswertes reagiert.

Im Sinne der vorliegenden Erfindung ist unter einer Prüfstoffmenge eine Menge eines Fluides in flüssiger, gasförmiger oder in flüssig- gasförmiger Phase zu verstehen, welches durch Verdunstung, Zerstäubung, Verneblung, Verdampfung oder mittels einer Druckveränderung, insbesondere durch eine Entspannung mittels Druckverminderung, zu einem Prüfgas im Sinne der vorliegenden Erfindung umwandelbar ist.

Die Bereitstellung einer erfindungsgemäßen Überprüfungsmöglichkeit, ob ein ungehinderter Gaszutritt durch das mindestens eine Gaszutrittselement in den Gassensor hinein gegeben ist, ermöglicht es dem Anwender, etwa dem Wartungspersonal, insbesondere in größeren Industrieanlagen, die Funktionsbereitschaft von Gassensoren ohne großen Personalaufwand an Zeit, insbesondere und vorzugsweise damit auch regelmäßig, durchzuführen.

Ein ungehinderter Gaszutritt ist die grundsätzliche Voraussetzung dafür, dass ein Ausgangssignal des Gassensors, welches eine unkritische Gaskonzentration, gleichsam als Nullsignal, - also eine vollständige oder nahezu vollständige Abwesenheit eines Schadgases, - korrekt vom Anwender auch als eine unkritische Situation interpretierbar ist. Problematisch ist eine Situation, in welcher das Nullsignal als Ausgangssignal des Gassensors ausgegeben wird, obwohl das mindestens eine Gaszutrittselement keinen ungehinderten Gaszutritt in den Gassensor hinein ermöglicht.

Erfindungsgemäß ist daher eine Einheit zur Prüfgasdosierung derart stromabwärts des mindestens einen Gaszutrittselements in der Anordnung zur Gasmessung angeordnet, welche ausgestaltet ist, eine flüssige, eine gasförmige oder eine Prüfstoffmenge in flüssig-gasförmiger Phase aus einem Prüfstoffvorrat (Behältnis, Prüfstoffreservoir, Prüfgasreservoir, Tank) zu einer in dem Gassensor angeordneten sensorisch-messtechnischen Anordnung hin zu dosieren oder zuströmen zu lassen. Die sensorisch-messtechnische Anordnung ist zu einer Erfassung einer Gaskonzentration oder einer Gaskonzentrationsänderung ausgebildet.
Unter einem Prüfstoffvorrat ist im Sinne der vorliegenden Erfindung ein Behältnis zur Bereithaltung und Bereitstellung der Prüfstoffmenge, also ein Fluid in flüssiger, gasförmiger oder in flüssig-gasförmiger Phase, zu verstehen. Der Prüfstoffvorrat ist als Tank, Prüfstoffreservoir, Prüfgasreservoir, Prüffluidreservoir oder Behälter (Flasche) ausgebildet, wobei die Prüfstoffmenge gasförmig unter Druck stehend oder in flüssigem Aggregatszustand bei Umgebungsdruck, wie auch in flüssig/ gasförmigen Zustandskombination, zumindest teilweise unter Druck stehend, wie beispielsweise in Form einer Flüssiggas-Flasche (Propan, Butan) in dem Prüfstoffvorrat bevorratet, gelagert und zur Dosierung mittels der Einheit zur Prüfgasdosierung bereitgestellt wird.

Als sensorisch-messtechnische Anordnungen in unterschiedlichen Typen von Gassensoren sind hier beispielhaft und nicht abschließend
- Elektroden/Elektrolyt-Kombinationen in elektrochemischen Gassensoren,
- Strahlungsquelle/Detektorelement-Kombinationen in Infrarotoptischen Gassensoren,
- katalytisch aktive und/ oder katalytisch passive Messelemente in katalytischen Gassensoren oder in Wärmetönungssensoren (Pellistoren),
- Feldeffekttransistoren mit gasartsensitiven Halbleiter-Elementen, beispielsweise gasartsensitiven Gate-Substraten in Halbleiter-Gassensoren
genannt.

Als Ausgestaltungsmöglichkeiten zur Realisierung von Einheiten zur Prüfgasdosierung sind beispielsweise Gasgeneratoren oder Ausgestaltungen des Prüfstoffvorrats oder Ausgestaltungen von Tankbehältnissen in Kombination mit Ventilen, Schaltmitteln oder Piezodosierelementen geeignet, welche von einer Kontrolleinheit derart betrieben, kontrolliert, gesteuert oder geregelt werden, dass definierte Mengen an Prüfgas oder Prüfstoffmenge dem Gassensor flüssig oder gasförmig zugeleitet oder zugeführt werden können.

Der Kontrolleinheit ist ein Datenspeicher zu einer Bereitstellung von vorbestimmten Zeitdauern, Messsignal-Schwellwerten, Vergleichskriterien oder Vergleichsgrößen zugeordnet oder der Datenspeicher ist als ein Element in der Kontrolleinheit mit enthalten.

Beispielsweise ermöglicht eine Dosierung mittels in Beginn und Ende von der Kontrolleinheit definierter Zeitintervalle oder Zeitdauern eine reproduzierbare und genaue Einstellung der Prüfstoffmenge, insbesondere dann, wenn diese Prüfstoffmenge im Prüfstoffvorrat unter einem definierten und bekannten Druck bevorratet ist.

Bevorzugt erfolgt die Zuführung der Prüfstoffmenge oder des Prüfgases mittels der Einheit zur Prüfgasdosierung in flüssiger Form in den Gassensor hinein, da eine Bevorratung oder Lagerung in flüssiger Form in dem Prüfstoffvorrat den Vorteil mit sich bringt, in einem relativ kleinen Volumen, nahe am Gassensor eine Prüfstoffmenge zu platzieren, welche für die Dauer der Einsatzzeit (Lebensdauer) der Gassensoren ausreichend ist. Eine solche Lagerung ist beispielsweise aus Gas-Feuerzeugen bekannt, bei denen Butan als Flüssiggas verwendet wird. Erfindungsgemäß weisen der Gassensor oder die Anordnung zur Gasmessung mindestens eine sensorisch-messtechnische Anordnung zu einer Erfassung einer Gaskonzentration oder einer Gaskonzentrationsänderung auf. Der Gassensor ist mittels der sensorisch-messtechnischen Anordnung geeignet ausgestaltet und dazu vorgesehen, aus einer Messumgebung mittels eines Gaseinlasses an diesen Gassensor herangeführte Gase oder Gasgemische qualitativ, wie auch quantitativ zu analysieren.

An dem Gassensor oder an der Anordnung zur Gasmessung sind zudem das mindestens eine Gaszutrittselement, die Einheit zur Prüfgasdosierung und die Kontrolleinheit angeordnet oder zugeordnet.
Das mindestens eine Gaszutrittselement ist an dem Gassensor am Gaseinlass oder an einem Gaseinlass der Anordnung zur Gasmessung derart angeordnet, dass Luft, Gase oder Gasgemische aus der Messumgebung zuvor das Gaszutrittselement passieren müssen, um zu der sensorisch-messtechnischen Anordnung im Gassensor zu gelangen. Vorzugsweise erfolgt die Zuführung durch das Gaszutrittselement mittels Diffusion. Das mindestens eine Gaszutrittselement ist dabei beispielsweise als eine semi- permeable oder permeable Membran, ein Schutzgitter oder eine Flammschutzscheibe, bzw. Sinterscheibe ausgestaltet und dient dabei funktional als Membran einerseits als Schutz gegen ein Eindringen von Verschmutzungen oder Feuchtigkeit in den Gassensor, wie auch in der Ausgestaltung als Flammschutzscheibe als Explosionsschutz. Dies mindestens eine Gaszutrittselement ist zur Entfaltung der zuvor beschriebenen funktionalen Wirkungen stromaufwärts der sensorisch-messtechnischen Anordnung angeordnet.

Eine Ausgestaltung des Gassensors oder der Anordnung zur Gasmessung oder der Gasmessvorrichtung mit mehreren Gaszutrittselementen ergibt sich beispielsweise in einer Konstellation mit mehreren Gassensoren in einer Anordnung zur Gasmessung oder Gasmessvorrichtung derart, dass jeder der mehreren Gassensoren mindestens ein an dem jeweiligen Gaseinlass des Gassensors direkt angeordnetes Gaszutrittselement, - beispielsweise als Membran ausgestaltet-, stromaufwärts der jeweiligen sensorisch-messtechnischen Anordnung aufweist und zudem an einem zentralen Gaseinlass der Anordnung zur Gasmessung oder Gasmessvorrichtung ein weiteres Gaszutrittselement, - beispielsweise als Schutzgitter oder Flammschutzscheibe ausgestaltetangeordnet ist.

Die Einheit zur Prüfgasdosierung kann in einer solchen Ausgestaltung erfindungsgemäß sowohl stromabwärts eines der direkt am jeweiligen Gaseinlass eines der Gassensoren direkt angeordneten Gaszutrittselementen angeordnet sein, als auch stromabwärts des weiteres Gaszutrittselements, also stromaufwärts eines der Gaseinlässe und deren zugehörigen Gaszutrittselemente der Gassensoren in der Anordnung zur Gasmessung. In beiden Fällen ist die Einheit zur Prüfgasdosierung im Verlauf der Zuströmung zwischen dem Gaszutrittselement oder Gaszutrittselementen und einer der sensorisch-messtechnischen Anordnungen in der Anordnung zur Gasmessung angeordnet. Die Strömungsrichtungen, - stromaufwärts, wie stromabwärts - sind durch die Zuströmung von Gas aus der Messumgebung in Richtung zum Gassensor oder in Richtung der Anordnung zur Gasmessung hin zu den sensorisch-messtechnischen Anordnungen definiert.

Das erfindungsgemäße Verfahren zur Überprüfung des mindestens einen Gaszutrittselements des Gassensors oder zur Überprüfung des mindestens einen Gaszutrittselements einer Anordnung zur Gasmessung mit mindestens einem Gassensor erfolgt gemäß einem ersten Aspekt der Erfindung mittels einer von einer Kontrolleinheit gesteuerten Abfolge von Betriebszuständen von einem Startzeitpunkt, beginnend mit einem ersten Betriebszustand mit einem kontinuierlichen Messbetrieb derart, dass:
- die Kontrolleinheit in einem zweiten Betriebszustand eine Dosierung einer Prüfstoffmenge mittels der, stromabwärts des Gaszutrittselements und stromaufwärts der sensorisch-messtechnischen Anordnung angeordneten, Einheit zur Prüfgasdosierung zu einer in dem Gassensor angeordneten sensorisch-messtechnischen Anordnung in dem Gassensor bewirkt,
- die Kontrolleinheit in einem dritten Betriebszustand einen Beginn eines Erwartungszeitfensters initiiert,
   die Kontrolleinheit mit dem Beginn des Erwartungszeitfensters mindestens ein Messsignal oder eine Vielzahl von Messsignalen des Gassensors erfasst und die Kontrolleinheit das mindestens eine erfasste Messsignal oder eines der Vielzahl von Messsignalen mit einem ersten Messsignal-Schwellwert vergleicht,
- die Kontrolleinheit in einem vierten Betriebszustand mindestens ein weiteres Messsignal oder eine weitere Vielzahl von Messsignalen des Gassensors erfasst und die Kontrolleinheit das mindestens eine weitere erfasste Messsignal oder eines der weiteren Vielzahl von Messsignalen mit einem zweiten Messsignal-Schwellwert vergleicht
   und die Kontrolleinheit ein Ende des Erwartungszeitfensters initiiert,
- die Kontrolleinheit in einem fünften Betriebszustand auf Basis des Vergleichs des Messsignals mit dem ersten Messsignal-Schwellwert und/ oder mit dem zweiten Messsignal-Schwellwert bestimmt, ob das Gaszutrittselement zu einer Zuführung von Luft, Gas oder Gasgemisch aus einer Messumgebung funktionsbereit ist und ein Maß für eine Funktionsbereitschaft des Gassensors und/oder der Anordnung zur Gasmessung mit mindestens einem Gassensor bestimmt.

Das erfindungsgemäße Verfahren ermöglicht es, aus dem kontinuierlichen Messbetrieb im ersten Betriebszustand heraus, in regelmäßigen Zeitintervallen, beispielsweise in einem in der Gasmesseinrichtung oder am Gassensor wählbaren Zeitrhythmus von 24 Stunden bis zu mehreren Tagen oder Wochen eine Überprüfung dahingehend vorzunehmen, ob ein ungehinderter Zutritt von Gas aus der Messumgebung zu der Gasmesseinrichtung oder zu dem Gassensor gewährleistet ist. Der kontinuierliche Messbetrieb stellt dabei gleichsam den regulären Betriebszustand der Gasmesseinrichtung, wie auch den regulären Betriebszustand des Gassensors dar und kann als eine kontinuierliche, fortlaufende oder regelmäßige Erfassung von Messsignalen, eine mittels einer Erfassungsrate (Abtastrate) zeitgesteuerte Erfassung von Messsignalen, beispielsweise mit einer Erfassungsrate, welche eine Messsignalerfassung einer vorbestimmten Anzahl von Messsignalen je Sekunde oder Minute entspricht, ausgestaltet sein oder als eine Messsignalerfassung ausgestaltet sein, wobei durch ein Ereignis oder einen Trigger die Erfassung einer vorbestimmten Anzahl von Messsignalen initiiert wird.
Die erfindungsgemäße Überprüfung des Zutritts aus der Messumgebung, bzw. der Gasdurchlässigkeit des Gaszutrittselements stellt dabei lediglich eine Unterbrechung des kontinuierlichen Messbetriebs für eine kurze Zeitdauer von im Regelfall weniger als einer Minute dar.

Der zweite Betriebszustand ist der Betriebszustand, in welchem durch die Dosierung der Prüfstoffmenge stromabwärts des Gaszutrittselements und stromaufwärts der sensorisch-messtechnischen Anordnung, also an einem Ort, welcher zwischen dem Gaszutrittselement und der sensorisch-messtechnischen Anordnung gelegen ist, beginnt. Die Dosierung erfolgt damit im Innern des Gassensors. Die an diesem Ort im Innern des Gassensors zu einem ersten Zeitpunkt t₁ dosierte Prüfstoffmenge gelangt dabei als Prüfgas als eine Prüfgas-Zuströmung zu der sensorisch-messtechnischen Anordnung hin und, im Falle, dass das Gaszutrittselement für das dosierte Prüfgas gasdurchlässig ist, durch das Gaszutrittselement als eine Prüfgas-Abströmung in die Messumgebung, also aus dem Gassensor wieder hinaus. Die Zeitdauer, beginnend mit der Dosierung der Prüfstoffmenge oder beginnend mit der Prüfgas-Zuströmung an die sensorisch-messtechnischen Anordnung für eine Zeitdauer, welcher Summe der Zeitdauern der Prüfgas-Zuströmung zur sensorisch-messtechnischen Anordnung und der Prüfgas-Abströmung in die Messumgebung entspricht, wird als Prozesszeitdauer bezeichnet.

Der dritte Betriebszustand ist ein Betriebszustand, welcher an die im zweiten Betriebszustand erfolgte Dosierung der Prüfstoffmenge und die mit der Dosierung oder Prüfgas-Zuströmung beginnende Prozesszeitdauer zeitlich anschließt oder die Prozesszeitdauer zumindest teilweise zeitlich überlappt und den Beginn eines Erwartungsfensters initiiert. Mit Beginn oder zu Anfang des Erwartungsfensters wird zu einem dritten Zeitpunkt t₃ mindestens ein Messsignal oder eine Vielzahl von Messsignalen erfasst und mit einem ersten Messsignal-Schwellwert verglichen.

Im vierten Betriebszustand wird im Zeitverlauf des Erwartungsfensters zu einem vierten Zeitpunkt t₄, vorzugsweise vor oder bei der Initiierung des Endes des Erwartungsfensters, mindestens ein weiteres Messsignal oder eine weitere Vielzahl von Messsignalen erfasst und mit einem zweiten Messsignal-Schwellwert verglichen. Der Beginn und das Ende des Erwartungsfensters, die Werte des ersten und zweiten Messsignalschwellwerts, wie auch die zeitliche Lage der Zeitpunkte zum Signalvergleich (dritter Zeitpunkt t₃, vierter Zeitpunkt t₄) im Erwartungsfenster sind jeweils vorzugsweise von einem zuvor ermittelten typischen Signalverhalten der messtechnischen Anordnung und Gegebenheiten (Menge, Zeitverhalten, Konzentration) der Dosierung der Prüfstoffmenge abgeleitet. Dabei gehen vorzugsweise das Ansprechverhalten (Anstiegszeit, z. B. t₁₀₋₉₀ - Anstiegszeit, Abfallzeit, z.B. t₉₀₋₁₀ - Abfallzeit), der Verlauf der Signalamplitude mit Nullsignal und maximaler Signalamplitude bei einer ungehinderten Zuströmung durch das Gaszutrittselement zu der messtechnischen Anordnung und ungehinderten Abströmung in die Messumgebung durch das Gaszutrittselement mit ein.
Dabei wird als Wert des zweiten Messsignalschwellwertes ein Wert mit geringerer Signalamplitude gewählt als der Wert des ersten Messsignalschwellwertes. Diese Dimensionierungen des ersten und zweiten Messsignalschwellwertes ergeben sich daraus, dass sich während des Erwartungsfensters bei einer ungehinderten Abströmung der dosierten Prüfstoffmenge aus dem Gassensor durch das Gaszutrittselement in die Messumgebung ein Signalabfall des Messsignals ergibt.

Im fünften Betriebszustand erfolgt die Überprüfung, ob das Gaszutrittselement zu einer Zuführung von Luft, Gas oder Gasgemisch aus der Messumgebung funktionsbereit ist. Wenn sowohl der Vergleich im dritten Betriebszustand, wie auch der Vergleich im vierten Betriebszustand ergibt, dass beide Messsignalschwellwerte unterschritten wurden, so ist ein Gasaustausch mit der Messumgebung möglich und daher das Gaszutrittselement zu einer Zuführung von Luft, Gas oder Gasgemisch aus der Messumgebung funktionsbereit. Die Kontrolleinheit bestimmt auf Basis der Vergleiche das Maß für die Funktionsbereitschaft des Gassensors und/oder der Anordnung zur Gasmessung mit mindestens einem Gassensor.

In einer besonderen Ausführungsform ist vorgesehen, dass für die Zeit der Unterbrechung die Gasmesseinrichtung oder der Gassensor ein Ersatzsignal bereitstellt. Dies dient dazu, mögliche Fehlinterpretationen der Messdaten auf einem Anzeigeelement oder an einer Datenschnittstelle, beispielsweise in Form einer Auslösung eines Gaskonzentrationsalarms, zu vermeiden. Ein solches Ersatzsignal kann beispielsweise ein zeitlich vorheriges Messsignal oder ein Signal sein, was von einem oder mehreren zeitlich vorherigen Messsignalen abgeleitet wird und welches mittels einer Ausgabeeinheit bereitgestellt wird. Als Ersatzsignal kann aber auch ein Statussignal von der Ausgabeeinheit bereitgestellt werden, welches für die Dauer der Überprüfung des Gaszutrittselements indiziert, dass die Gasmesseinrichtung oder der Gassensor aktuell nicht zur Messung von Gasen aus der Messumgebung bereit sind.

In einer bevorzugten Ausführungsform wird in dem zweiten Betriebszustand von der Kontrolleinheit die Dosierung der Prüfstoffmenge von der Einheit zur Prüfgasdosierung zu der sensorisch-messtechnischen Anordnung in dem Gassensor derart bewirkt, dass die Kontrolleinheit im Zeitverlauf zu einem ersten Zeitpunkt (Aktivierungszeitpunkt) t₁ die Einheit zur Prüfgasdosierung aktiviert. Diese Aktivierung ist beispielsweise als ein Einschalten eines Ventils mit einem elektrischen Steuersignal ausgestaltet.

Vorzugsweise wird in dieser bevorzugten Ausführungsform die Dosierung der Prüfstoffmenge als Dosierung in Form einer flüssigen Prüfstoffmenge bewirkt. Diese flüssig dosierte Prüfstoffmenge wechselt dann im Gassensor nach der Dosierung, beispielsweise durch eine Verdunstung oder durch einen Aufprall auf einer im/am Gassensor angeordneten Wandung bewirkte Zerstäubung in die gasförmige Phase über.

Eine Ausgestaltungsmöglichkeit der Dosierung im zweiten Betriebsschritt ist durch eine Realisierung der Einheit zur Prüfgasdosierung als ein Piezo-Dosierelement in Kombination mit dem Prüfstoffvorrat gegeben.
Ein solches Piezo-Dosierelement ist ausgestaltet, bei einer einmaligen Aktivierung eine genau definierte Prüfstoffmenge zu dosieren. Die Dosierung der genau definierten Prüfstoffmenge erfolgt dabei ohne, dass eine Deaktivierung zur Einstellung oder dosierten Prüfstoffmenge erforderlich ist. Ein Piezo-Dosierelement arbeitet nach dem piezoelektrischen Effekt, wobei bei Aktivierung durch einen elektrischen Spannungsimpuls eine Verformung im Piezo-Material, beispielsweise Keramik, bewirkt wird. Aus der Verformung resultiert ein, mit dieser Verformung korrespondierender Auspressdruck. Die definierte Prüfstoffmenge wird mit dem Auspressdruck durch eine feine Düse dosiert. Der Einsatz solcher Piezo-Dosierelemente, oder Piezo-Keramik-Dosierelemente ist beispielsweise aus dem Gebiet der Drucktechnik (Tintenstrahldrucker) als sogenannte Ink-Jet-Technologie bekannt.

Eine weitere alternative Ausgestaltungsmöglichkeit der Dosierung im zweiten Betriebsschritt ist durch eine Realisierung der Einheit zur Prüfgasdosierung als Kombination eines zur Verdampfung der flüssigen Prüfgasmenge geeigneten Heizelementes mit dem Prüfstoffvorrat gegeben. Mittels des Heizelementes ist mit einer einmaligen Aktivierung (Heizimpuls) eine Erzeugung einer definierten Prüfstoffmenge möglich. Als Beispiel für eine geeignete flüssige Prüfstoffmenge sei hier Aceton oder Ethanol genannt. Die Dosierung der genau definierten Prüfstoffmenge erfolgt dabei mittels einer explosionsartigen Bildung einer Dampfblase, ohne, dass eine Deaktivierung zur Einstellung der dosierten Prüfstoffmenge erforderlich ist. Der Einsatz solcher Heizelemente ist beispielsweise aus dem Gebiet der Drucktechnik (Tintenstrahldrucker) unter dem Begriff Bubble-Jet bekannt.

Wird beispielsweise zum Zeitpunkt t₁ in dem zweiten Betriebszustand eine flüssige Prüfstoffmenge, beispielsweise an Butan oder an Propan dosiert, so verdampft die flüssige Prüfstoffmenge und steht zu einem Zeitpunkt t_{1'} als eine gasförmige Prüfstoffmenge an der sensorisch-messtechnischen Anordnung im Gassensor zur Verfügung. Diese gasförmige Prüfstoffmenge bewirkt im Gassensor eine Reaktion der sensorisch-messtechnischen Anordnung in Form einer Änderung des Gaskonzentrationsmesswertes als Ausgangssignal.
Ist der Gassensor beispielsweise als ein elektrochemischer Gassensor ausgestaltet, so ergibt sich aufgrund chemischer und/oder elektrochemischer Reaktionen in/an der sensorisch-messtechnischen Anordnung eine Änderung des Messsignals oder Ausgangssignals.
Ist der Gassensor beispielsweise als ein Infrarot- optischer Gassensor mit einer Messküvette ausgestaltet, so ergibt sich in einem Wellenlängenbereich des optischen Gassensors bedingt durch die Absorptionseigenschaften des Prüfgases eine Bedämpfung der Lichtausbreitung in der Messküvette zu der sensorisch-messtechnischen Anordnung eine Änderung des Ausgangssignals.
Ist der Gassensor beispielsweise als ein katalytischer Gassensor ausgestaltet, so ergibt sich aufgrund von Verbrennungsreaktionen und/oder chemischen Reaktionen am Messelement (Pellistor) als sensorisch-messtechnische Anordnung mit der gasförmigen Prüfstoffmenge eine Änderung des Ausgangssignals.
Ist der Gassensor beispielsweise als ein Halbleiter Gassensor ausgestaltet, so ergibt sich aufgrund chemischer Reaktionen an den gasartsensitiven Halbleiter-Elementen der sensorisch-messtechnischen Anordnung eine Änderung des Ausgangssignals.

In einer bevorzugten Ausführungsform wird im dritten Betriebszustand von der Kontrolleinheit die Dosierung der Prüfstoffmenge von der Einheit zur Prüfgasdosierung zu der sensorisch-messtechnischen Anordnung in dem Gassensor derart bewirkt, dass die Einheit zur Prüfgasdosierung zu einem ersten Zeitpunkt (Aktivierungszeitpunkt) t₁ aktiviert und zu einem zweiten Zeitpunkt (Deaktivierungszeitpunkt) t₂ deaktiviert wird.
Die Einheit zur Prüfgasdosierung dosiert für ein vorbestimmtes Zeitintervall (Δt_{Dose} = t₂ - t₁) bis zum zweiten Zeitpunkt (Deaktivierungszeitpunkt) t₂ eine - mittelbar durch das Zeitintervall - vorbestimmte flüssige Prüfstoffmenge zur sensorisch-messtechnischen Anordnung in den Gassensor.

Der zweite Zeitpunkt (Deaktivierungszeitpunkt) t₂ ergibt sich als ein von dem ersten Zeitpunkt (Aktivierungszeitpunkt) t₁ zeitlich beabstandeter Zeitpunkt durch eine Deaktivierung der Einheit zur Prüfgasdosierung mittels der Kontrolleinheit. Eine Ausgestaltung mit Aktivierung und Deaktivierung ist beispielsweise in vorteilhaften Ausgestaltungen von elektrischen Schaltsignalen in Verbindung mit der Einheit zur Prüfgasdosierung mit einem oder mehreren elektrisch gesteuerten Ventilen ideal und praktikabel.

Die vorbestimmte Prüfstoffmenge wird durch das vorbestimmte Zeitintervall in Verbindung mit den vorgegebenen Randbedingungen, wie dem im Prüfstoffvorrat herrschenden Druck, Temperatur und einem Öffnungsquerschnitt eines zur Aktivierung und Deaktivierung eingesetzten Ventils bestimmt. Eine solche vorbestimmte flüssige Prüfstoffmenge wird auch Bolus oder Bolusmenge genannt. Als Ventile sind verschiedenste zur Dosierung von Fluiden oder Flüssigkeiten verwendbare Ventiltypen, wie digitale Schaltventile mit binären Zuständen (Normally Open [NO] oder Normally Closed [NC]) oder auch Proportionalventile in der Einheit zur Prüfgasdosierung einsetzbar.

Eine weitere Ausgestaltung mit Aktivierung und Deaktivierung ist beispielsweise in Form von elektrischen Einstellsignalen für eine Gaserzeugung mittels eines oder mehrerer Gasgeneratoren als Einheit zur Prüfgasdosierung praktikabel.

In einer besonderen Ausführungsform wird von der Kontrolleinheit eine Größe und/oder ein Volumen des Gassensors für die Bestimmung des zweiten Zeitpunktes (Deaktivierungszeitpunkt) t₂ im Zeitverlauf t berücksichtigt. Die Berücksichtigung der Größe und/oder des Volumens des Gassensors ergibt die Möglichkeit, dass unterschiedliche Größen oder Volumina von Gassensoren, welche unterschiedliche Einströmverhältnisse und Einströmmengen bedingen, berücksichtigt werden können. Auf diese Weise kann durch die Wahl des zweiten Zeitpunktes (Deaktivierungszeitpunkt) t₂ eine an Größe und Volumen angepasste Prüfstoffmenge von der Einheit zur Prüfgasdosierung mittels der Kontrolleinheit dosiert werden.

In einer weiteren bevorzugten Ausführungsform wird von der Kontrolleinheit eine Größe und/oder Volumen des Gassensors bei der Bestimmung der Menge der flüssigen Prüfstoffmenge, wie auch bei der Dosierung der Menge der flüssigen Prüfstoffmenge mittels der Einheit zur Prüfgasdosierung berücksichtigt. In dieser Ausführungsform wird die Menge der flüssigen Prüfstoffmenge korrespondierend zu der Größe und/oder dem Volumen des Gassensors angepasst, es wird ein angepasster Bolus der Prüfstoffmenge, welche mit der Größe und/oder dem Volumen des Gassensors korrespondiert von der Kontrolleinheit bestimmt und mittels der Einheit zur Prüfgasdosierung dosiert.

In einer weiteren bevorzugten Ausführungsform wird in dem dritten Betriebszustand von der Kontrolleinheit der dritte Zeitpunkt t₃ von dem ersten Zeitpunkt (Aktivierungszeitpunkt) t₁ im Zeitverlauf oder von einem von diesem ersten Zeitpunkt (Aktivierungszeitpunkt) t₁ abgeleiteten Zeitpunkt t_{1'} abgeleitet. Der erste Zeitpunkt (Aktivierungszeitpunkt) t₁ ist für die Dosierung der flüssigen Prüfstoffmenge zu der sensor-messtechnischen Anordnung im zweiten Betriebszustand repräsentativ. Die Ableitung des dritten Zeitpunkts t₃ von dem ersten Zeitpunkt (Aktivierungszeitpunkt) t₁ erfolgt derart, dass der dritte Zeitpunkt t₃ für die Prozesszeitdauer von dem ersten Zeitpunkt t₁ oder dem Zeitpunkt t_{1'} derart zeitlich beabstandet ist, dass die Prozesszeitdauer einer Zeitdauer entspricht, in welche eine ungehinderte Zuströmung aus der Messumgebung durch das Gaszutrittselement zu der sensorisch-messtechnischen Anordnung in den Gassensor hinein und einer anschließenden ungehinderten Ausströmung aus dem Gassensor in die Messumgebung wieder hinaus entspricht oder mit dieser Prozesszeitdauer korrespondierend ist.

In einer besonders bevorzugten Ausführungsform wird von der Kontrolleinheit eine Größe und/oder ein Volumen des Gassensors, eine Anzahl, eine Dicke, eine Porengröße, eine Fläche und/oder ein Durchmesser des Gaszutrittselements für die Bestimmung der zeitlichen Dauer der Prozesszeitdauer und/oder für den dritten Zeitpunkt t₃ im Zeitverlauf t berücksichtigt.

In einer besonders bevorzugten Ausführungsform wird von der Kontrolleinheit eine Größe und/oder ein Volumen des Gassensors, eine Anzahl, eine Dicke, eine Fläche und/oder ein Durchmesser des Gaszutrittselements für die Bestimmung der zeitlichen Dauer des Erwartungszeitfensters und/oder für die Bestimmung des vierten Zeitpunkts t₄ im Zeitverlauf t berücksichtigt. Diese Berücksichtigung der Größe und/oder des Volumens des Gassensors, wie auch der Anzahl, der Dicke, der Porengröße, der Fläche und/oder des Durchmessers des Gaszutrittselements bei der Bestimmung der Prozesszeitdauer oder bei der Bestimmung der zeitlichen Dauer des Erwartungszeitfensters ermöglicht es, die Überprüfung des Gassensors an die jeweilige Gassensor-Konstellation in der Anordnung zur Gasmessung oder Gasmessvorrichtung anzupassen, da Anzahl, Dicke, Fläche oder Durchmesser, Porengröße des Gaszutrittselements die Geschwindigkeit des Gaszutritts durch Diffusion wie auch des Gasaustritts durch Diffusion wesentlich mit bestimmen.

In einer weiteren bevorzugten Ausführungsform des Verfahrens wird von der Kontrolleinheit auf Basis des Vergleichs im fünften Betriebszustand ein Zustandssignal ermittelt und/oder bereitgestellt. Der Vergleich im fünften Betriebszustand, liefert das Resultat, ob die eindosierte Prüfstoffmenge innerhalb des Erwartungszeitfensters aus dem Gassensor durch das Gaszutrittselement wieder hinausgeströmt, bzw. hinausdiffundiert ist. Falls die Diffusion aus dem Gassensor, bzw. die Ausströmung aus dem Gassensor nicht innerhalb der zeitlichen Dauer des Zeitfensters erfolgt ist, so kann von der Kontrolleinheit ein Zustandssignal generiert werden, welches anzeigt, dass keine korrekte Funktionsbereitschaft des Gassensors oder der Anordnung zur Gasmessung gegeben ist.

Aus der Situation, dass eine eindosierte Prüfstoffmenge nicht erwartungsgemäß dem Gassensor durch das Gaszutrittselement wieder verlassen hat, kann festgestellt werden, dass auch die Zuströmung aus der Messumgebung in den Gassensor hinein durch das Gaszutrittselement nicht optimal gegeben oder ggf. gar durch eine Blockade unmöglich ist.

In einer weiteren bevorzugten Ausführungsform wird das Zustandssignal von der Kontrolleinheit an die Ausgabeeinheit, ein zentrales Auswertesystem, eine Alarmzentrale oder ein mobiles Ausgabemittel bereitgestellt. Ein zentrales Auswertesystem fungiert dabei als eine Gaswarnanlage, welche Gasalarme einer Vielzahl von Gassensoren (Transmitter) oder Gasmessvorrichtungen an einer zentralen Stelle empfängt, bereitstellt, visualisiert und alarmiert, wie auch eine Kontrolle der Vielzahl der Gassensoren oder Gasmessvorrichtungen, der Daten- und Versorgungsverbindungen zu der Vielzahl der Gassensoren oder Gasmessvorrichtungen koordiniert. Eine solche Bereitstellung an eine Ausgabeeinheit, ein zentrales Auswertesystem oder eine Gaswarnanlage, eine Alarmzentrale oder ein mobiles Ausgabemittel, wie beispielsweise ein Mobiltelefon oder ein mobiles anderes Kommunikationsmittel, ermöglicht es, den Zustand des Gassensors oder der Anordnung zur Gasmessung an weitere externe Stellen mitzuteilen. Dazu sind geeignete Datenleitungen oder Datenübertragungsmittel (0-10V, 4- 20 mA, Ethernet, LAN, W-LAN, USB, RS232, etc.) zur Verbindung der Kontrolleinheit mit der Ausgabeeinheit wie auch dem zentralen Auswertesystem, dem Gaswarnsystem oder der Alarmzentrale oder den mobilen Ausgabemitteln vorgesehen. Die Datenleitungen sind ausgestaltet, Messdaten, wie auch Gaskonzentrationswerte, Alarmsignale oder auch Störsignale, an die Ausgabeeinheit, das zentrale Auswertesystem, das Gaswarnsystem, die Alarmzentrale oder mobile Ausgabemittel zu übertragen.

In einer weiteren Ausführungsform erfolgt von der Ausgabeeinheit, dem zentralen Auswertesystem, dem Gaswarnsystem, der Alarmzentrale oder den mobilen Ausgabemitteln eine Ausgabe eines Alarmsignals oder eine Ausgabe einer Meldung.

In einer weiteren bevorzugten Ausführungsform wird das Alarmsignal von der Kontrolleinheit und/oder der Ausgabeeinheit einem akustischen Alarmgeber zu einer akustischen Alarmierung und/oder einem optischen Signalgeber zu einer optischen oder visuellen sichtbaren Alarmierung bereitgestellt.

In einer weiteren bevorzugten Ausführungsform wird die bereitgestellte Meldung auf einem Datensichtgerät, einem Bildschirm oder einem anderen zu einer Visualisierung geeigneten Gerät als ein Hinweis, insbesondere als ein Warnhinweis oder als eine Anweisung in Textform, graphischer Form oder in symbolischer Form sichtbar von der Kontrolleinheit und/oder der Ausgabeeinheit bereitgestellt.

Die beschriebenen Ausführungsformen stellen jeweils für sich als auch in Kombination miteinander besondere Ausgestaltungen des erfindungsgemäßen Verfahrens zur Überprüfung des Gassensors oder der Gasmessvorrichtung, insbesondere des Gaszutrittselementes, dar. Dabei sind sich durch Kombination oder Kombinationen mehrerer Ausführungsformen ergebende Vorteile und weitere Ausführungsformen gleichwohl vom Erfindungsgedanken mit erfasst, wenn auch nicht sämtliche Kombinationsmöglichkeiten von Ausführungsformen dazu im Detail jeweils ausgeführt sind. Die vorstehend beschriebenen Ausführungsformen des Verfahrens können auch in Form eines computerimplementierten Verfahrens als Computerprogrammprodukt mit einem Computer ausgebildet sein, wobei der Computer zur Durchführung des oben beschriebenen, erfindungsgemäßen Verfahrens veranlasst wird, wenn das Computerprogramm oder Teile des Computerprogramms auf dem Computer bzw. auf einem Prozessor des Computers oder einem sogenannten "Embedded System" als Teil einer Gasmessvorrichtung, Gassensors oder Anordnung zur Gasmessung oder einem der Gasmessvorrichtung, dem Gassensor oder der Anordnung zur Gasmessung zugeordneten - vorzugsweise computerunterstützten- Auswertesystem oder Gaswarnsystem ausgeführt wird.

Dabei kann das Computerprogramm auch auf einem maschinenlesbaren Speichermedium gespeichert sein. In einer alternativen Ausgestaltung kann ein Speichermedium vorgesehen sein, welches zur Speicherung des vorstehend beschriebenen, computerimplementierten Verfahrens bestimmt ist und von einem Computer lesbar ist. Es liegt im Rahmen der vorliegenden Erfindung, dass alle Schritte des Verfahrens oder des Computerprogramms nicht zwangsläufig auf ein und derselben Computerinstanz ausgeführt werden müssen, sondern sie können auch auf unterschiedlichen Computerinstanzen ausgeführt werden. Auch kann die Abfolge der Verfahrensschritte gegebenenfalls variiert werden.

Vorstehend wurde die Lösung der Aufgabe in Bezug auf das, als ersten Aspekt der Erfindung, beanspruchte Verfahren beschrieben. Hierbei erwähnte Merkmale, Vorteile oder alternative Ausführungsformen sind ebenso auch auf die, nach einem weiteren Aspekt der Erfindung beanspruchten Gegenstände zu übertragen und umgekehrt. Die entsprechenden funktionalen Merkmale des Verfahrens werden dabei durch entsprechende gegenständliche Module oder Einheiten einer Vorrichtung, insbesondere durch Hardware-Bausteine (µC, DSP, MP, FPGA, ASIC, GAL), ausgebildet, die beispielsweise in Form eines Prozessors, mehrere Prozessoren (µC, µP, DSP) oder in Form von Instruktionen in einem Speicherbereich implementiert sein können, die durch den Prozessor verarbeitet werden.

Damit ergibt sich ein weiterer Aspekt der Erfindung, der die gestellten Aufgaben erfindungsgemäß durch eine Vorrichtung zur Durchführung des Verfahrens zur Überprüfung eines Gassensors und Gasmessvorrichtung mit einer Prüfvorrichtung zur Überprüfung des Gassensors löst. Diese zur Durchführung des Verfahrens geeignete Vorrichtung ist derart ausgestaltet, die Überprüfung des Gassensors und/oder der Gasmessvorrichtung oder Anordnung zur Gasmessung gemäß den in dem Verfahren beschriebenen Schritten durchzuführen, sowie auch die in den Ausführungsformen beschriebenen weiteren Schritte jeweils für sich oder in Kombination auszuführen.

Die erfindungsgemäße Gasmessvorrichtung mit einer Prüfvorrichtung zur Durchführung des Verfahrens weist dazu mindestens einen Gassensor mit einem Gaszutrittselement, mit mindestens einer sensorisch-messtechnischen Anordnung, eine Einheit zur Prüfgasdosierung, eine Kontrolleinheit und einen der Kontrolleinheit zugeordneten Datenspeicher auf. Die Verarbeitung und die Steuerung der Betriebszustände erfolgt mittels der Kontrolleinheit. Die Steuerung der Messsignal-Erfassung, die Steuerung der Dosierung, die Speicherung von Messsignalen, die Vergleiche der Messsignale mit Schwellwerten und die Bestimmung des Maßes für die Funktionsbereitschaft des Gassensors wird dabei von der Kontrolleinheit - teilweise in Verbindung mit dem Datenspeicher - vorgenommen. In dem Gassensor oder der Anordnung zur Gasmessung ist das Gaszutrittselement stromaufwärts der sensorisch-messtechnischen Anordnung angeordnet und die Einheit zur Prüfgasdosierung ist stromabwärts des Gaszutrittselementes in dem Gassensor oder der Anordnung zur Gasmessung angeordnet.

In einer Ausführungsform ist die Einheit zur Prüfgasdosierung als ein Piezodosierelement und ein mit dem Piezodosierelement fluidisch verbundenen Reservoir ausgestaltet. Das Reservoir ist, beispielsweise als ein Tank, zu einer Lagerung oder einer Bevorratung eines Prüfstoffvorrates ausgebildet. Die Kontrolleinheit ist zu einer Aktivierung des Piezodosierelementes zu einem ersten Zeitpunkt t₁ ausgebildet, um eine Dosierung einer Prüfstoffmenge, welche in dem Prüfstoffvorrat bevorratet ist, zu dem Gassensor oder zu der Anordnung zur Gasmessung zu bewirken.

In einer bevorzugten Ausführungsform ist die Einheit zur Prüfgasdosierung durch ein Ventil in Verbindung mit einem, mit dem Ventil fluidisch verbundenen Reservoir ausgebildet. Das Reservoir ist, beispielsweise als Tank, zu einer Lagerung oder Bevorratung eines Prüfstoffvorrates ausgebildet. Die Kontrolleinheit ist ausgebildet, eine Aktivierung des Ventils zu einem ersten Zeitpunkt t₁ und eine Deaktivierung des Ventils zu einem zweiten Zeitpunkt t₂ zu bewirken, um eine Dosierung einer Prüfstoffmenge aus dem Prüfstoffvorrat zu dem Gassensor oder der Anordnung zur Gasmessung zu bewirken. Aktivierung und Deaktivierung erfolgen dadurch vorzugsweise mittels elektrischer Schaltsignale, welche von der Kontrolleinheit an die Einheit zur Prüfgasdosierung übermittelbar sind.

In einer besonders bevorzugten Ausführungsform weist die Gasmessvorrichtung eine Ausgabeeinheit, einen optischen Alarmgeber oder einen akustischen Alarmgeber auf. Der optische Alarmgeber und/oder der akustische Alarmgeber sind in Zusammenwirkung mit der Kontrolleinheit und/oder der Ausgabeeinheit zu einer Ausgabe eines Alarmsignals ausgebildet und vorgesehen. Dabei weist die Gasmessvorrichtung zusätzlich eine optionale Schnittstelle auf, welche in Zusammenwirkung mit der Kontrolleinheit zu einer Übermittlung eines Zustandssignals an ein Auswertesystem ausgebildet und vorgesehen ist.

In einer weiteren bevorzugten Ausführungsform ist die sensorische und messtechnische Anordnung als eine Kombination von Elektroden und einem Elektrolyten in einem elektrochemischen Gassensor, als eine Kombination einer Strahlungsquelle und eines Detektorelements in einem infrarotoptischen Gassensor, als eine Kombination von katalytisch aktiven und/oder katalytisch passiven Messelementen in einem katalytischen Gassensor, oder in einem Wärmetönungssensor, sowie als Gasart spezifische und sensitive Halbleiterelemente in einem Halbleitergassensor ausgestaltet.

Die vorliegende Erfindung stellt mit dem Verfahren zur Überprüfung eines Gassensors und mit der Gasmessvorrichtung mit einer Prüfvorrichtung zur Überprüfung eines Gassensors eine vorteilhafte und technisch sehr gut realisierbare Möglichkeit bereit, auf sichere Art und Weise festzustellen, ob ein ungehinderter Gaszutritt durch das Gaszutrittselement in die Gasmessvorrichtung oder zu dem Gassensor möglich ist.

Daneben ergibt sich als ein weiterer Vorteil, dass auch die Funktion des Gassensors selbst, wie auch die Dosierung der Prüfstoffmenge selbst mittels der Methodik der Dosierung der vorbestimmten Prüfstoffmenge mit überprüfbar ist. Das ergibt sich daraus, dass das Messsignal auch dann keine Änderung zeigt, wenn, entweder die sensorisch- messtechnischen Elemente im Gassensor nicht ordnungsgemäß funktionieren, bzw. defekt sind, oder aber, wenn die Dosierung der vorbestimmten Prüfstoffmenge nicht ordnungsgemäß funktioniert.
Damit ergibt sich der Vorteil, dass mit dem Verfahren zur Überprüfung eines Gassensors und mit der Gasmessvorrichtung mit einer Prüfvorrichtung zur Überprüfung eines Gassensors neben der Überprüfung des dem Gassensor oder der Gasmessvorrichtung zugehörigen Gaszutrittselements, sowohl die Prüfvorrichtung mit der Funktion der Dosierung der Prüfstoffmenge selbst, als auch die Funktion des Gassensors oder der Gasmessvorrichtung, bzw. der sensorisch-messtechnischen Elemente, wie auch von Elementen zur Signalverarbeitung überprüfbar sind. Damit ergibt insgesamt der Vorteil, dass im Wesentlichen keine mögliche Situation unbemerkt bleibt, in welcher eine Fehlfunktion einer oder mehrerer der wesentlichen für einen verlässlichen, sicheren und qualitativ hochwertigen Betrieb erforderlichen Komponenten des Gassensors oder der Anordnung zur Gasmessung mit einem Gassensor, also Gaszutrittselement oder der sensorisch-messtechnischen Elemente, wie auch der Elemente (Zuleitungen, Elektronik, Verstärker, A/D- Wandler) zur Signalverarbeitung auftritt.

Ein besonderer Vorteil ergibt sich zusätzlich, dass auch eine mögliche Fehlfunktion der Einheit zur Prüfgasdosierung, wie beispielweise eine mögliche Blockade oder Leckage bei der Zuführung der Prüfstoffmenge oder des zur Dosierung angeordneten Ventils, eine Störung in der elektrischen Steuerung des Ventils (z.B. Signalfehler, Leitungsbruch, Drahtbruch) oder ein leerer Prüfstoffvorrat ebenfalls erkennbar ist. Diese Fehlerursachen sind dabei nicht in allen Fällen voneinander abgrenzbar und unterscheidbar, für den Einsatz ergibt sich der Vorteil, dass im Wesentlichen funktionsfähige Anordnungen zur Gasmessung und Gassensoren von Anordnungen und Gassensoren mit Funktionsstörungen klar unterscheidbar sind.

Gemäß einem weiteren Aspekt der Erfindung, der die gestellte Aufgabe erfindungsgemäß durch ein Gaswarnsystem mit mindestens einem Gassensor oder mit einer Gasmessvorrichtung löst, wird die Überprüfung der Funktionsbereitschaft des Gaszutrittselements des Gassensors oder der Gasmessvorrichtung von dem Gaswarnsystem koordiniert. Das Gaswarnsystem aktiviert dabei die Prüfvorrichtung zur Überprüfung der Gasmessvorrichtung hinsichtlich der Funktionsbereitschaft des Gaszutrittselements.

In der Gasmessvorrichtung ist eine Einheit zur Prüfgasdosierung angeordnet, wie in den Ausführungsformen der erfindungsgemäßen Gasmessvorrichtung gemäß dem einen weiteren Aspekt der Erfindung beschrieben. Geeignete Ausgestaltungsmöglichkeiten einer Einheit zur Prüfgasdosierung sind beispielsweise Gasgeneratoren oder Ausgestaltungen des Prüfstoffvorrats oder Ausgestaltungen von Tankbehältnissen in Kombination mit Ventilen, Schaltmitteln oder Piezodosierelementen, welche derart betrieben, kontrolliert, gesteuert oder geregelt werden, dass definierte Prüfstoffmengen dem Gassensor flüssig oder gasförmig zugeleitet oder zugeführt werden können. Dabei wird eine Dosierung der Prüfstoffmenge stromabwärts des Gaszutrittselements und stromaufwärts der sensorisch-messtechnischen Anordnung ermöglicht, wie in den Ausführungsformen der erfindungsgemäßen Gasmessvorrichtung gemäß dem einen weiteren Aspekt der Erfindung beschrieben.

Das Gaswarnsystem koordiniert die Überprüfung des Gassensors und/oder der Gasmessvorrichtung in einer Weise, wobei der Ablauf der Überprüfung des Gaszutrittselements in der Gasmessvorrichtung derart erfolgt, wie zu dem erfindungsgemäßen Verfahren gemäß dem ersten Aspekt der Erfindung beschriebenen Schrittabfolge und auch zu den Ausführungsformen jeweils beschriebenen weiteren Schritten und deren möglichen Kombinationen.

Die Aktivierung der Prüfvorrichtung, wie auch der Empfang von Messsignalen erfolgt mittels der im Zusammenhang mit der Bereitstellung des Zustandssignals genannten Datenleitungen oder Datenübertragungsmittel (0- 10V, 4- 20 mA, Ethernet, LAN, W-LAN, USB, RS232, etc.).

Die Gasmessvorrichtung befindet sich im üblichen Betrieb in einem ersten Betriebszustand in einem kontinuierlichen Messbetrieb, in welchem die Gasmessvorrichtung regelmäßig Messwerte der in der Gasmessvorrichtung angeordneten Gassensoren und Zustandswerte der Gasmessvorrichtung über die Datenübertragungsmittel an das Gaswarnsystem übermittelt. Das Gaswarnsystem koordiniert die Überprüfung des Gassensors oder der Gasmessvorrichtung in beginnend aus dem kontinuierlichen Messbetrieb heraus in folgender Weise:
- Deaktivierung einer Ausgabe von diesem Gassensor oder dieser Gasmessvorrichtung zugehörigen Alarmierungen und/oder Störungsmeldungen in dem Gaswarnsystem
- Aktivierung einer Dosierung einer Prüfstoffmenge mittels der Einheit zur Prüfgasdosierung zu einer in dem Gassensor angeordneten sensorisch-messtechnischen Anordnung,
- Initialisierung einer vorbestimmten Wartezeit, wobei die Dauer der Wartezeit einer Zeitdauer entspricht, in welcher die eindosierte Prüfstoffmenge in den Gassensor durch das Gaszutrittselement hinein und anschließend aus dem Gassensor durch ein funktionsbereites Gaszutrittselement wieder hinausgeströmt, bzw. hinausdiffundiert ist,
- Reaktivierung der Ausgabe von diesem Gassensor oder dieser Gasmessvorrichtung zugehörigen Alarmierungen und/oder Störungsmeldungen in dem Gaswarnsystem nach Ablauf der vorbestimmten Wartezeit.

Bei oder während der Deaktivierung der Ausgabe von diesem Gassensor oder dieser Gasmessvorrichtung zugehörigen Alarmierungen in dem Gaswarnsystem kann die Überprüfung der Funktionsbereitschaft des Gaszutrittselements durchgeführt werden, eine Alarmierung findet am Gaswarnsystem dabei nicht statt.

Während der Wartezeit erfolgt die Überprüfung der Funktionsbereitschaft des Gaszutrittselements gemäß dem ersten Aspekt der Erfindung mit der Abfolge von Betriebszuständen beginnend aus einem ersten Betriebszustand (kontinuierlicher Messbetrieb) als Startzeitpunkt mit dem zweiten Betriebszustand (Prüfgas-Eindosierung), dritten Betriebszustand (Initialisierung Erwartungszeitfenster, Messsignalerfassung), vierten Betriebszustand (Messsignalerfassung, Schwellenwertvergleich) bis zum fünften Betriebszustand (Bestimmung der Funktionsbereitschaft).

In einer bevorzugten Ausführungsform kann an dem Gaswarnsystem vorgesehen sein, den aktuellen Zustand oder Fortschritt der Überprüfung der Funktionsbereitschaft des Gaszutrittselements kenntlich zu machen, auszugeben oder zu signalisieren. Dies kann beispielsweise mittels einer besonderen Blinkfolge von optischen Alarmgebern (LED) am Gaswarnsystem oder auch mittels einer Textausgabe auf einem alphanumerischen Anzeigeelement (Display) erfolgen.

Bei oder nach der Reaktivierung der Ausgabe von diesem Gassensor oder dieser Gasmessvorrichtung zugehörigen Alarmierungen in dem Gaswarnsystem nach Ablauf der vorbestimmten Wartezeit ergeben sich folgende zwei Konstellationen:

### Konstellation 1:

Die eindosierte Prüfstoffmenge ist während der vorbestimmten Wartezeit nach der Eindosierung durch das Gaszutrittselement wieder hinausgeströmt, bzw. hinausdiffundiert. Das Gaszutrittselement ist also funktionsbereit, d.h. nicht verschmutzt oder in der Gasdurchlässigkeit vermindert. Die von der Gasmessvorrichtung oder dem Gassensor erfassten Messwerte indizieren dann einen Zustand, in welchem keine erfassbare Menge eines Messgases oder Gasgemisches im Gassensor und in der Messumgebung vorhanden ist, auf welches der mindestens eine Gassensor auf eine Änderung einer Gaskonzentration dieses Messgases empfindlich ist mit Änderungen des Gaskonzentrationsmesswertes reagiert. Es wird am Gaswarnsystem keine Alarmierung ausgegeben, da der Zustand evaluiert ist, dass sowohl das Gaszutrittselement gasdurchlässig und damit funktionsbereit ist, wie auch, dass kein Messgas in einer, eine Alarmierung indizierenden Konzentration von dem Gassensor oder der Gasmessvorrichtung erfasst worden ist.

### Konstellation 2:

Die eindosierte Prüfstoffmenge ist während der vorbestimmten Wartezeit nach der Eindosierung durch das Gaszutrittselement nicht wieder hinausgeströmt, bzw. hinausdiffundiert. Das Gaszutrittselement ist also nicht funktionsbereit, d.h. beispielweise verschmutzt und daher in der Gasdurchlässigkeit vermindert.
Die von der Gasmessvorrichtung oder dem Gassensor erfassten Messwerte indizieren dann einen Zustand, in welchem eine erfassbare Menge eines Messgases oder Gasgemisches im Gassensor und in der Messumgebung vorhanden ist, auf welches der mindestens eine Gassensor auf eine Änderung einer Gaskonzentration dieses Messgases empfindlich ist mit Änderungen des Gaskonzentrationsmesswertes reagiert. Es wird am Gaswarnsystem eine Alarmierung ausgegeben, da der Zustand evaluiert ist, dass Messgas in einer, eine Alarmierung indizierenden Konzentration von dem Gassensor oder der Gasmessvorrichtung erfasst worden ist.

Da die Überprüfung durch das Gaswarnsystem selbst zuvor initiiert worden ist, ist es gemäß einer bevorzugten Ausführungsform vorgesehen, dass in dem Gaswarnsystem bei der Ausgabe der Alarmierung der Zustand, dass zuvor die Initiierung der Überprüfung von dem Gaswarnsystem veranlasst worden ist, berücksichtigt worden ist. Dies kann beispielsweise derart erfolgen, dass keine Ausgabe eines Gasalarmes, welche eine Anwesenheit einer einen vorbestimmten Schwellenwert übersteigenden Messgaskonzentration indiziert, zur Ausgabe verwendet und bereitgestellt wird, sondern eine Ausgabe erfolgt, welche indiziert, dass eine Funktionsbereitschaft des Gassensors oder der Gasmessvorrichtung, insbesondere eine Funktionsbereitschaft des Gassensors oder der Gasmessvorrichtung nicht gegeben ist. Eine solche Ausgabe kann beispielsweise mittels einer besonderen Blinkfolge von optischen Alarmgebern (LED) am Gaswarnsystem oder auch mittels einer Ausgabe eines Hinweises auf einem alphanumerischen Anzeigeelement (Display) erfolgen.

Diese Art der Ausgabe am Gaswarnsystem ergibt den Vorteil, dass außer für die Initiierung der Prüfung und die Deaktivierung und Reaktivierung der Ausgabe keine weiteren Anpassung in der Zusammenwirkung zwischen Gasmessvorrichtung und Gaswarnsystem erforderlich ist. Die bereits in jedem Alarmierungen auf Basis von erhöhten Konzentration eines Messgases an einzelnen oder mehreren Gasmessvorrichtungen werden dazu mit verwendet, Zustände von Funktionsbereitschaften einzelner oder mehrere Gaszutrittselemente auszugeben. Zu einer Unterscheidung von Messgasalarmierungen aufgrund von erhöhten Messgaskonzentrationen in der Messumgebung einer bestimmten Gasmessvorrichtung braucht dazu in dem Gaswarnsystem lediglich Information aus dem laufenden Betrieb des Gaswarnsystems mit verwendet werden, nämlich die, dass das Gaswarnsystem unmittelbar vor der Übermittlung von erhöhten Messgas- Konzentrationen durch diese bestimmte Gasmessvorrichtung, an dieser bestimmten Gasmessvorrichtung eine Überprüfung gemäß dem weiteren Aspekt der Erfindung initiiert hat.

Die vorliegende Erfindung wird mit Hilfe folgender Figuren und den zugehörigen Figurenbeschreibungen ohne Beschränkung des allgemeinen Erfindungsgedankens näher erläutert.

Es zeigen in schematischer oder vereinfachter Darstellung:
- Fig. 1a: Eine Anordnung zur Gasmessung mit einem optischen Gassensor und einer Prüfvorrichtung,
- Fig. 1b: Eine Anordnung zur Gasmessung mit einem katalytischen Gassensor und einer Prüfvorrichtung,
- Fig. 1c: Eine Anordnung zur Gasmessung mit einem elektrochemischen Gassensor und mit einer Prüfvorrichtung,
- Fig. 1d: Eine Anordnung zur Gasmessung mit einem Halbleiter- Gassensor,
- Fig. 2: Einen typischen Verlauf eines Messsignals eines Gassensors während einer Überprüfung mit der Prüfvorrichtung,
- Fig. 3: Einen Ablauf zur Überprüfung des Gassensors mit der Prüfvorrichtung.

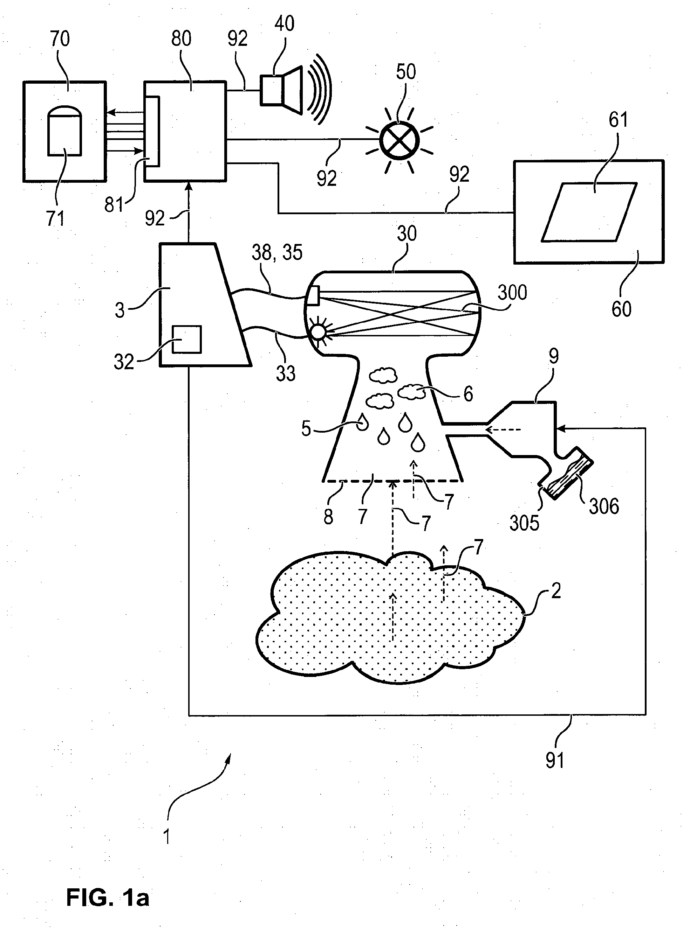

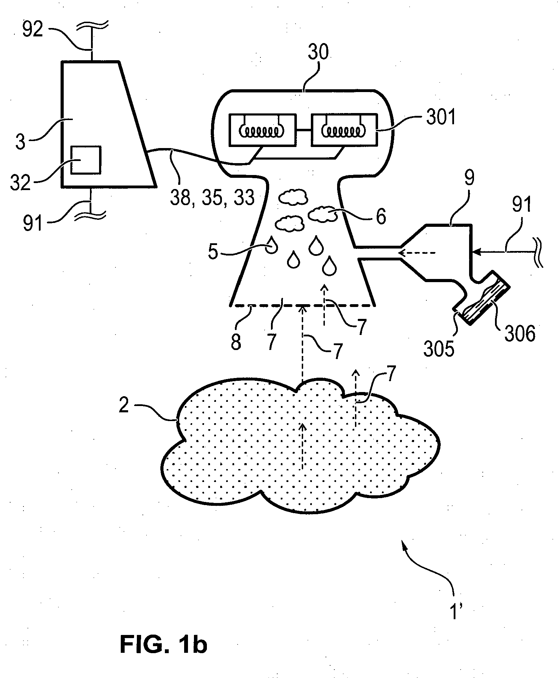

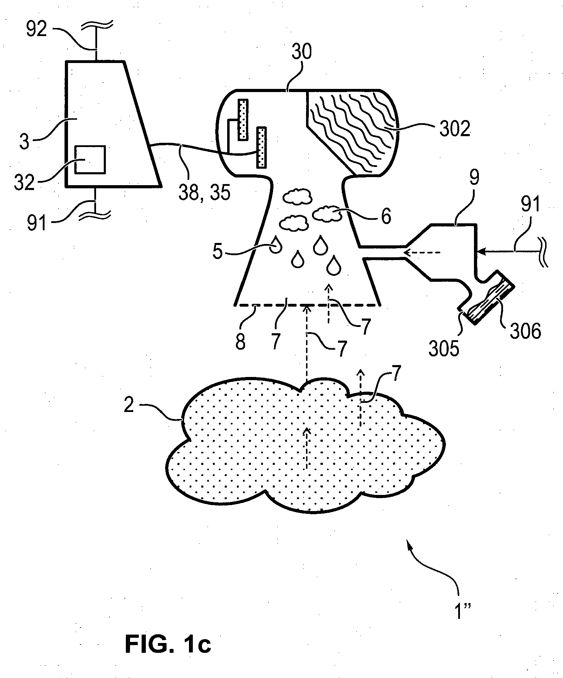

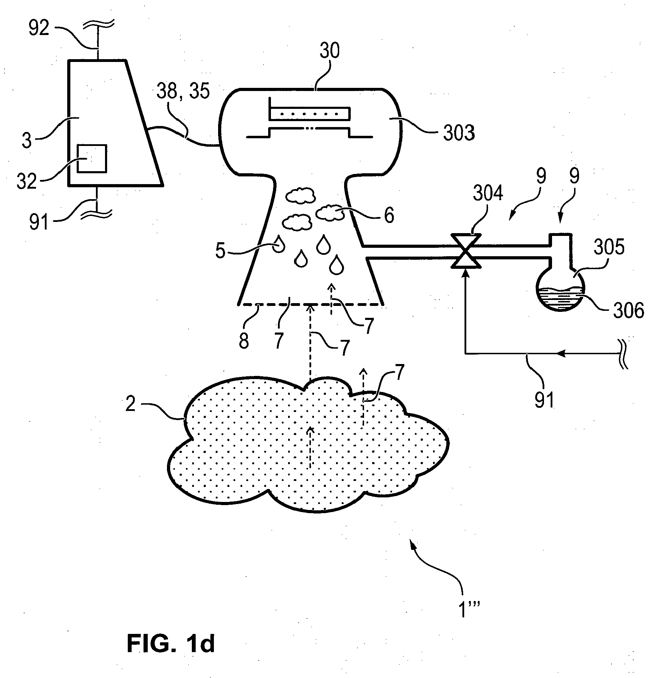

Die Figuren 1 a, 1 b, 1 c sowie 1 d zeigen Anordnungen zur Gasmessung mit einem Gassensor und mit einer Prüfvorrichtung. In der Figur 1a ist eine Anordnung 1 zur Gasmessung mit einem optischen Gassensor 300 gezeigt. Der optische Gassensor 300 ist als eine Küvette mit einer, -in dieser Figur 1 a aus Gründen der zeichnerischen Übersichtlichkeit nicht näher im Detail dargestellten -Multireflexionszelle ausgebildet. In der Multireflexionszelle sind als sensorisch - messtechnische Anordnung eine Strahlungsquelle und ein Detektorelement angeordnet. In der Multireflexionszelle wird Licht von der Strahlungsquelle auf eine gegenüberliegende Wandung, sowie seitliche Wandungen abgestrahlt, von dort reflektiert und nach mehrfacher Reflexion vom Detektorelement erfasst. Eine Anwesenheit eines zu messenden Prüfgases, beispielsweise Methan, Ethan, Butan, Propan verändert die Absorptionseigenschaften für das abgestrahlte Licht im infraroten Wellenbereich. Dies ist als Messeffekt eines abgeschwächten Signals auf dem Detektorelement erfassbar. Somit ergibt sich der Messeffekt einer Dämpfung des abgestrahlten IR-Lichtes durch bestimmte Gase, beispielsweise Methan, Ethan, Butan, Propan und andere Kohlenwasserstoffe. Das Gas aus einer Messumgebung 2 gelangt in den optischen Gassensor 300 über ein Gaszutrittselement 8, beispielsweise eine semi-permeable oder permeable Membran, ein Schutzgitter oder eine Flammschutzscheibe in die Messküvette des optischen Gassensors 300 hinein. In dieser Figur 1a ist als Ausgestaltung des Gaszutritts 7 mit einem Gaszutrittselement 8 lediglich ein Gaszutrittselement 8 gezeigt.

In einer Ausgestaltung einer Anordnung zur Gasmessung 1 in einem Gerät, in welchem mehrere Gassensoren als Gassensorik 30 angeordnet sind, ist es in vielen technischen Ausgestaltungen technisch üblich und vorteilhaft, mehrere Gaszutrittselemente 8 in Reihe hintereinander angeordnet vorzusehen. So ist es vorstellbar, dass von der Messumgebung 2 zwei aus stromabwärts ein erstes Gaszutrittselement als Flammschutz oder Staubschutz fungiert, anschließend ein zweites Element einen Zutritt von Feuchtigkeit verhindert und ein drittes Element 8 im eigentlichen Gassensor, beispielsweise den optischen Gassensor 300 oder einen katalytischen 301 (Fig. 1b) oder einen elektrochemischen Gassensor 302 (Fig. 1 c) oder einen Halbleitergassensor 303 (Fig. 1 d) schützt. Die Anordnung der Einheit zur Prüfgasdosierung 9 kann dabei sowohl stromabwärts von der Messung aus zwischen dem ersten und zweiten Gaszutrittselement, zwischen dem zweiten und dritten Gaszutrittselement 8 oder zwischen dem dritten Gaszutrittselement 8 und der Gassensorik 30, 300 erfolgen. Diese Ausgestaltungen mit mehreren Gaszutrittselementen und möglichen, geeigneten Positionen der Anordnung der Einheit zur Prüfgasdosierung 9 sind aus Gründen der Übersichtlichkeit in dieser Figur 1 a in der Anordnung zur Gasmessung 1 nicht mit gezeigt. Diese Ausgestaltungsmöglichkeiten sind aber im Sinne der vorliegenden Erfindung als Anordnungen der Einheit zur Prüfgasdosierung 9 an der Gassensorik 30 mit - umfasst. Ein solcher Gaszutritt 7 erfolgt aus einer Messumgebung 2 hin zum optischen Gassensor 300.

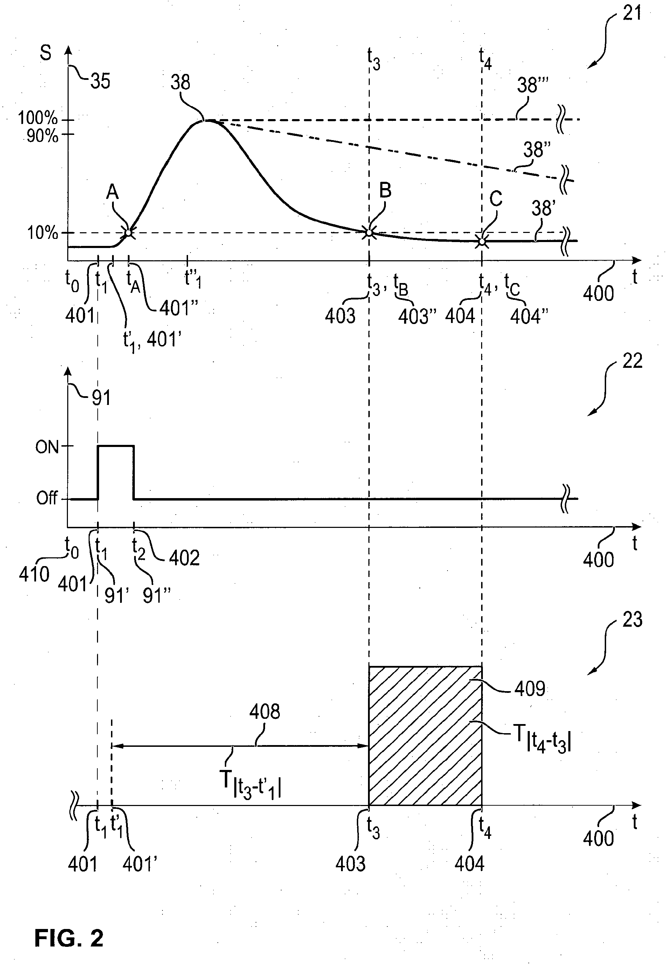

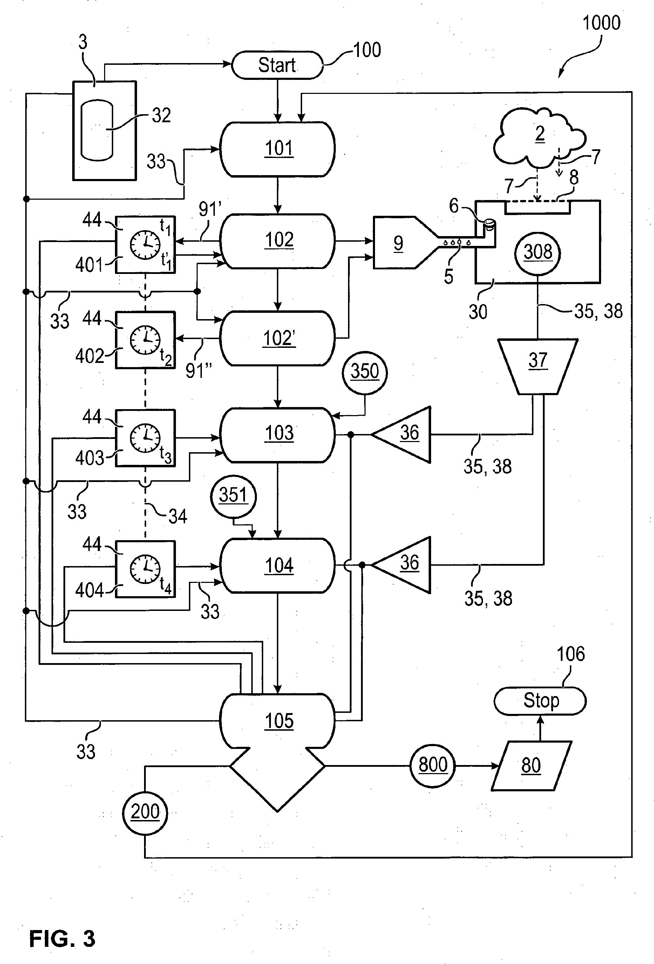

In dieser Anordnung zur Gasmessung 1 nach Figur 1 a ist an den optischen Gassensor 300 stromabwärts zum Gaszutrittselement 8 eine Einheit zur Prüfgasdosierung 9 angeordnet. Von dieser Einheit zur Prüfgasdosierung 9 wird aus einem Prüfstoffvorrat 305, beispielsweise aus einem Tank 305 mit einer Vorratsmenge 306 eine flüssige Prüfstoffmenge 5 dosiert. Diese flüssig eingespritzte Prüfstoffmenge 5 verdunstet, zerstäubt oder verdampft in dem Gassensor 300 zu einer gasförmigen Prüfstoffmenge 6, welche dann zur Messung in dem optischen Gassensor 300 befindlich ist. Die Einheit zur Prüfgasdosierung 9 wird mittels einer Steuerleitung 91 von einer Kontrolleinheit 3 derart angesteuert, dass zu vorbestimmten Zeitpunkten t₁ eine vorbestimmte Menge flüssiger Prüfstoffmengen 5 stromabwärts des Gaszutrittselements 8 in den optischen Gassensor 300 eindosiert wird. In einer bevorzugten Variante ist die Einheit zur Prüfgasdosierung 9 als ein Piezo-Dosierelement ausgeführt. Ein solches Piezo-Dosierelement ist in Kombination mit dem Prüfstoffvorrat 305 ausgestaltet, bei einer einmaligen Aktivierung mittels eines Steuersignals 91' (Figuren 2, 3) jeweils eine genau definierte Prüfstoffmenge zu dosieren, ohne, dass eine Deaktivierung des Piezo-Dosierelementes, beispielsweise durch ein weiteres Steuersignal 91 "(Figuren 2, 3) oder durch eine genau definierte Zeitdauer des Steuersignals durch eine Zeitsteuerung 44 (Figur 3) erforderlich ist.
Die Kontrolleinheit 3 nimmt Messsignale 35, 38 vom optischen Gassensor 300, bzw. vom Detektorelement in dem optischen Gassensor 300 auf. Weiterhin steuert die Kontrolleinheit 3 den infrarot optischen Strahler in dem optischen Gassensor 300 mittels einer Kontrollleitung 33. Das Messsignal 35, wie auch ein auf dem Messsignal 35 basierender Messsignalverlauf 38 werden von der Kontrolleinheit 3 mittels einer Daten- oder Signalleitung 92 an eine Ausgabeeinheit 80 übergeben. Die Ausgabeeinheit 80 ist ausgebildet, mittels Signal- und Datenleitung 92 einen akustischen Alarmgeber, beispielsweise einer Hupe 40, oder optischen Alarmgeber, beispielsweise eine Lampe 50, anzusteuern. Weiterhin ist die Ausgabeeinheit 80 mittels einer Schnittstelle 81 dazu ausgebildet, über Signal- und Datenleitungen 92, wie auch Steuerleitungen 91, Daten, Auswerteergebnisse, Sensorsignale, Datensignale oder aufbereitete Messsignale 35, 38 an ein Auswertesystem 70 weiterzugeben. In dem Auswertesystem 70 ist vorzugsweise eine Datenbank 71 angeordnet, welche Zustände und Ergebnisse von Überprüfungen der Anordnung zur Gasmessung 1 protokollieren kann. Von der Ausgabeeinheit 80 ist über eine Signal- und Datenleitung 92 eine Bedien- und Darstellungseinheit (User Interface) 60 angebunden. Die Bedien- und Darstellungseinheit 60 weist einen Bildschirm 61 auf, auf welchen Fehlermeldungen sowie Hinweise an einen Anwender, wie auch Messsignale oder Messwerte darstellbar sind. Die Kontrolleinheit 3 und die Einheit zur Prüfgasdosierung 9 wirken, in Verbindung mit einem in der Kontrolleinheit 3 angeordneten Datenspeicher 32 oder einem der Kontrolleinheit 3 zugeordneten Datenspeicher 32, zusammen in einem Verfahren zur Überprüfung der Anordnung der Gasmessung 1, wie in Figur 2 und 3 näher erläutert wird. Dabei wird die Reaktion des optischen Gassensors 300 auf eine Dosierung einer flüssigen Prüfstoffmenge 5 mit Verdunstung von der flüssigen Prüfstoffmenge 5 in eine gasförmige Prüfstoffmenge 6 in den optischen Gassensor 300 hin dazu verwendet, zu überprüfen, in welcher Zeit diese dosierte Prüfstoffmenge 5 über das Gaszutrittselement 8 wieder aus dem optischen Gassensor 300 hinausdiffundiert wird. Dazu wird von der Kontrolleinheit 3 ein Ablauf zur Überprüfung, wie genauer aus Figur 3 ersichtlich, durchgeführt. Mittels einer Zeitmessung wird festgestellt, ob die eindosierte Menge 5, 6 nach einer bestimmten Zeit wieder aus dem optischen Gassensor 300 entwichen ist, oder aber nicht. Ist nach einer vorbestimmten Zeit diese eindosierte Prüfstoffmenge 5, 6 nicht aus dem optischen Gassensor 300 entwichen, so kann von der Kontrolleinheit 3 gefolgert, oder bestimmt werden, dass am Gaszutrittselement 8 eine fehlerhafte Situation besteht.

Die Figur 1 b zeigt eine gegenüber der Figur 1 a abgewandelte Anordnung zur Gasmessung 1'. In der Figur 1 b ist anstelle des optischen Gassensors mit einer infraroten Multireflexionszelle 300 ein katalytischer Gassensor 301 gezeigt. Ein solcher katalytischer Gassensor 301, auch als Wärmetönungssensor bekannt, ist in ähnlicher Weise wie in Figur 1a zu dem optischen Gassensor 300 beschrieben mit einer Kontrolleinheit 3 und einer Einheit zur Prüfgasdosierung 9 verbunden. Gleiche Elemente in den Figuren 1 a und 1 b sind in den Figuren 1 a und 1 b mit den gleichen Bezugsziffern bezeichnet.

Die Beschreibung zur Funktionalität und Zusammenwirkung der Kontrolleinheit 3 mit der Einheit zur Prüfgasdosierung 9 ist, wie zu Figur 1 a ausgeführt, auch auf die Funktionalität der Zusammenwirkung von Kontrolleinheit 3 und katalytischem Gassensor 301 mit Einbeziehung der Einheit zur Prüfgasdosierung 9 zur Überprüfung des Gaszutrittselements 8 übertragbar.

Die in der Figur 1 a gezeigten Elemente Ausgabeeinheit 80, Auswertesystem 70 und Bedien- und Darstellungseinheit 60 mit den entsprechenden weiteren Elementen, sowie die mit diesen Elementen verbundenen Datenleitungen 92, sowie Steuerleitungen 91 sind in der Figur 1 b nicht im Detail mit gezeigt. Es ist aber im Sinne der vorliegenden Erfindung mit umfasst, dass die Anordnung zur Gasmessung 1' in ähnlicher Weise mit Auswertesystem 70, Auswerteeinheit 80 und der Bedien- und Darstellungseinheit 60 zusammenwirken kann, wie zu Figur 1 a zur Anordnung zur Gasmessung 1 ausgeführt. Die Steuerleitungen 91 und Datenleitungen 92 sind in dieser Anordnung zur Gasmessung 1' lediglich vereinfacht schematisch angedeutet.

Im Unterschied zur Figur 1 a mit der Anordnung zur Gasmessung 1 ergibt sich in dieser Anordnung zur Gasmessung 1' in dieser Figur 1 b der Aspekt, dass eine andere Gassensorik 30, ausgestaltet als ein oder zwei katalytische Messelemente als sensorisch-messtechnische Anordnung in dem katalytischen Gassensor 301 angeordnet ist, welche bei Zuführung eines besonderen Gases, beispielsweise Ethan, Methan, Butan oder Propan, eine chemische Reaktion mit diesem Gas eingehen. Bei dieser Reaktion an den katalytischen Messelementen wird ein Teil des Gases verbraucht. Dies hat den Effekt, dass eine eindosierte Prüfstoffmenge 5, 6 nicht vollständig aus dem katalytischen Gassensor 301 nach einer vorbestimmten Zeit durch das Gaszutrittselement 8 wieder in die Messumgebung 2 entweicht, sondern, dass es eine Mindermenge gibt, welche durch den Verbrauch an Messgas durch die katalytisch aktiven Messelemente in dem katalytischen Gassensor 301 bedingt ist. Dieser Effekt ist bei der Überprüfung der Anordnung zur Gasmessung 1', bzw. der Überprüfung des Gaszutrittselements 8 mittels der eindosierten, bzw. eindiffundierten Prüfstoffmengen 5, 6 und den wieder entwichenen Gasmengen nach einer bestimmten Zeit zu berücksichtigen. Dies ist in der Figur 2 und der zugehörigen Beschreibung näher erläutert.

In der Figur 1c ist eine Anordnung zur Gasmessung 1" mit einem elektrochemischen Gassensor 302 gezeigt. Gleiche Elemente in den Figuren 1 a, 1b und 1c sind mit den gleichen Bezugsziffern in den Figuren 1 a, 1b und 1 c bezeichnet. Im Unterschied zur Figur 1 a mit der Anordnung zur Gasmessung 1 ergibt sich in dieser Anordnung zur Gasmessung 1" in dieser Figur 1 c der Aspekt, dass eine andere Gassensorik 30, ausgestaltet als sensorisch-messtechnische Anordnung eines vorzugsweise flüssigen Elektrolyten und einer Anordnung von Elektroden in dem elektrochemischen Gassensor 302 angeordnet ist. Bei einer bei Zuführung eines besonderen Gases, beispielsweise Ammoniak, erfolgt eine elektrochemische Reaktion oder chemische Umsetzung an den Elektroden. Die Anordnung zur Gasmessung 1" ist in ähnlicher Weise vereinfacht dargestellt, wie die Anordnung zur Gasmessung 1' nach Figur 1 b. Zu dieser vereinfachten Darstellung ausgeführte Anmerkungen in der Beschreibung zu Figur 1 b sind auf diese Figur 1c übertragbar. Hinsichtlich der Zusammenwirkung der Kontrolleinheit 3 mit der Einheit zur Prüfgasdosierung 9 und dem elektrochemischen Gassensor 302 ist anzumerken, dass auch eine Gassensorik 30 mit einem elektrochemischen Gassensor 302, ähnlich wie ein katalytischer Gassensor 301 nach Figur 1b eine gewisse Menge des Prüfgases während der Messung, bedingt durch die chemische Umsetzung an den Elektroden, verbraucht. Auch in dieser Ausgestaltung nach Figur 1 c ist dies bei der Überprüfung bzw. Bilanzierung der eindosierten Prüfstoffmengen 5, 6 und der wieder durch das Gaszutrittselement 8 entweichenden Gasmengen zu berücksichtigen. Dies wird in der Figur 2, bzw. in der Beschreibung zu Figur 2 näher ausgeführt.

In der Figur 1d ist eine Anordnung zur Gasmessung 1'" gezeigt. Diese Anordnung zur Gasmessung 1'" weist einen Halbleitergassensor 303 als Gassensorik 30 auf. Die zu den Figuren 1a, 1b und 1c gemachten Ausführungen sind entsprechend auch auf die Figur 1 d anwendbar. Die Anordnung zur Gasmessung 1'" ist vergleichbar zur Figur 1b und 1 c in einer vereinfachten Darstellung ausgeführt. Gleiche Elemente in den Figuren 1 a, b, 1 c und 1 d sind in den Figuren 1 a, b, 1 c und 1 d mit den gleichen Bezugsziffern versehen. In der Figur 1 d ist als Gassensorik 30 der Halbleitergassensor 303 gezeigt. Als weiterer Unterschied ist die Einheit zur Prüfgasdosierung 9 als eine Zusammenwirkung aus einem Prüfstoffvorrat 305 mit einem Ventil 304 gezeigt. Dies Ventil 304 wird mittels Steuerleitung 91 von der Kontrolleinheit 3 betätigt. In dem Prüfstoffvorrat 305 ist ähnlich wie in den Figuren 1 a, 1b, 1c zuvor beschrieben, eine Vorratsmenge 306 enthalten. Vorzugsweise handelt es sich in dieser Figur 1d bei der Vorratsmenge 306 um ein Flüssiggas, welches unter Druck stehend in dem Prüfstoffvorrat 305 enthalten ist. Bei Öffnung des Ventils 304 für einen vorbestimmten Zeitraum kann eine Menge der Vorratsmenge 306 von dem Prüfstoffvorrat 305 zu dem Halbleitergassensor 303 gelangen. Je nach Ausgestaltung des Überdrucks in dem Prüfstoffvorrat 305 gelangt eine Menge der Vorratsmenge 306 als flüssige Prüfstoffmenge 305 oder bereits verdunstete Prüfstoffmenge 6 zu dem Halbleitergassensor 303. Der Übergang von der flüssigen Phase der Prüfstoffmenge 5 zur gasförmigen Phase der Prüfstoffmenge 6 kann dabei unmittelbar durch Druck-Entspannung bei Öffnung des Ventils 304, wie auch bei einem Auftreffen oder Aufprall von flüssigen Prüfstoffmengen 5 auf Wandungen des Halbleitergassensors 303 erfolgen. Die Zusammenwirkung der Kontrolleinheit 3 mit der Einheit zur Prüfgasdosierung 9 bzw. Ventil 304 und Prüfstoffvorrat 305 ist in der Beschreibung zu Figur 2, sowie in dem dargestellten Ablauf nach Figur 3 und der dazugehörigen Beschreibung des Ablaufs nach Figur 3 näher im Detail erläutert.

Die Figur 2 zeigt einen typischen Verlauf eines Messsignals einer Anordnung zur Gasmessung mit einem Gassensor während einer Überprüfung mit einer Prüfvorrichtung. Es sind drei Diagramme 21, 22, 23 gezeigt, welche jeweils einen Zeitverlauf t 400 miteinander synchronisiert darstellen.

In einem ersten Diagramm 21 wird ein Messsignalverlauf 38, 38' und 38" als Zeitverlauf eines Messsignals S 35 dargestellt. Das Messsignal S 35 ist auf der Ordinate skaliert. Die Skalierung ist hervorgehoben bei 10%, 90% und 100%. Im Signalverlauf 38 sind die Signalwerte eingezeichnet, in denen das Messsignal 38 einen Wert von 10% der Messsignalamplitude S 35 von 100% erreicht. Diese Punkte sind im Verlauf 38 mit A, bzw. B gekennzeichnet.
Die dargestellten prozentualen Werte 10%, 90%, 100% sind für eine Veranschaulichung und Verdeutlichung des Signalverhaltens von Gassensoren vorteilhaft, da zeitliche und dynamische Eigenschaften von Gassensoren auf diese Weise vereinheitlicht beschrieben werden können und damit vergleichbar sind.
In der Messtechnik ist die sogenannte t₁₀₋₉₀ - Anstiegszeit geeignet und zur Charakterisierung eines Signalanstiegs auf eine Erhöhung einer Gaskonzentration auf 100% des maximalen Messbereichsumfangs gebräuchlich.
Die sogenannte t₁₀₋₉₀ - Anstiegszeit wird oftmals auch als Sensor- Ansprechzeit bezeichnet. Die t₁₀₋₉₀ - Anstiegszeit entspricht der Zeitdauer, während derer die Messsignale, - in einer Signal- Anstiegsphase-, Werte in einem Wertebereich zwischen 10% bis 90% des durch die Gaskonzentrationsänderung bedingten Endwertes (100%) des Signals aufweisen.
Zu einer Charakterisierung eines Signalabfalls auf eine Reduzierung oder Verminderung einer Gaskonzentration auf 10% des Messbereichsumfangs ergibt sich dazu die sogenannte t₉₀₋₁₀ - Abfallzeit in vergleichbarer Weise.
Die t₉₀₋₁₀ - Abfallzeit entspricht der Zeitdauer, während derer die Messsignale, - in einer Signal- Abfallphase-, Werte in einem Wertebereich zwischen 90% und 10% aufweisen.

Im zweiten Diagramm 22 ist der Verlauf eines Steuersignals 91 über dem Zeitverlauf t 400 gezeigt. Das Steuersignal 91 wird von der Kontrolleinheit 3 (Figuren 1a, 1b, 1c und 1d, 3) erzeugt.

Das dritte Diagramm 23 zeigt einen Ausschnitt aus dem Zeitverlauf t 400. In diesem Ausschnitt aus dem Zeitverlauf t 400 sind besonders wesentliche Zeitpunkte hervorgehoben, welche für die Überprüfung der Anordnung zur Gasmessung 1, 1', 1", 1'" (Figuren 1a, 1b, 1c und 1d) wesentlich sind. Der Zeitverlauf t 400 der wesentlichen Zeitpunkte in den drei Diagrammen 21, 22, 23 wird nun beginnend mit einem Zeitpunkt t₀ 410 beschrieben. Die gleich bezeichneten Zeitpunkte in den drei Diagrammen 21, 22, 23 sind zueinander jeweils zeitlich synchron. Der Zeitverlauf t 400 beginnt mit einem Zeitpunkt t₀ 410, bei dem die Anordnung zur Gasmessung 1, 1', 1", 1'" (Figuren 1a, 1b, 1c und 1d) Umgebungsluft aus der Messumgebung 2 (Figuren 1a, 1b, 1c und 1d) erfasst.

Das Messsignal S 35 zeigt zum Zeitpunkt t₀ 410 ein Grundsignal, dass die Abwesenheit von Prüfgas oder Schadgas repräsentiert.

Zu einem Zeitpunkt t₁ 401 wird von der Kontrolleinheit 3 (Figuren 1a, 1b, 1c und 1d) ein Schaltsignal 91' an die Einheit zur Prüfgasdosierung 9 (Figuren 1a, 1b, 1c und 1d) geschickt, um eine Prüfstoffmenge 5 (Figuren 1a, 1b, 1c und 1d) zu dem Gassensor 300, 301, 302, 303 (Figuren 1a, 1b, 1c und 1d) als flüssige Menge zu dosieren oder einzuspritzen. Zum Zeitpunkt t_{1'} 401' ist die Prüfstoffmenge 5 (Figuren 1a, 1b, 1c und 1d) in eine gasförmige Prüfstoffmenge 6 (Figuren 1 a, 1b, 1c und 1d) verdampft und steht damit als ein Prüfgas zur sensorisch-messtechnischen Erfassung durch die Gassensorik 30 (Figuren 1a, 1b, 1c und 1d) zur Verfügung. Zu einem, zeitlich nach dem Zeitpunkt t_{1'} liegenden Zeitpunkt t_{1"} kann anhand des Signalanstiegs 38 des Messsignals 35 in diesem Beispiel sicher davon ausgegangen werden, dass gasförmige Prüfstoffmengen zu der sensorisch-messtechnischen Anordnung (Figuren 1a, 1b, 1c und 1d) gelangt ist. Zum Zeitpunkt t₂ 402 wird die Einheit zur Prüfgasdosierung 9 (Figuren 1a, 1b, 1c und 1d) durch ein weiteres Steuersignal 91' wieder deaktiviert, so dass keine weitere Prüfstoffmenge 5 (Figuren 1a, 1b, 1c und 1d) an die Gassensorik 30 (Figuren 1a, 1b, 1c und 1d) hin dosiert wird. Das Messsignal S 35 reagiert auf die Eindosierung der Prüfstoffmenge 6 (Figuren 1a, 1b, 1c und 1d) mit einem Signalanstieg. Der Signalanstieg entspricht der aktuellen Veränderung der Gaskonzentration, verursacht durch die eindosierte gasförmige Prüfstoffmenge 6 (Figuren 1a, 1b, 1c und 1d). Zum Zeitpunkt t_{A}, 401" weist das Messsignal S 35 bereits 10% der Ausgangsamplitude auf. Der weiter folgende Signalanstieg 38 erreicht ungefähr an dem Zeitpunkt t_{1"} einen Amplitudenwert von 90%. Zwischenzeitlich wurde mittels des Steuersignals 91" die Dosierung der Prüfstoffmenge 5 (Figuren 1a, 1b, 1c und 1d) beendet. Im Fall eines ungehinderten Ein- und Ausströmens durch das Gaszutrittselement 8 (Figuren 1a, 1b, 1c und 1d) hindurch sinkt im Verlauf das Messsignal S 35. Zum Zeitpunkt t₃ 403 unterschreitet in dieser Darstellung nach der Figur 2 das Messsignal S 35 eine Amplitude von 10%. Dieser Amplitudenwert von 10% ist als Punkt B zu einem Zeitpunkt t_{B} 403" im Zeitverlauf 400 eingetragen. Zum Zeitpunkt t₄ 404, bzw. t_{C} 404" mit einem korrespondierenden Wert des Messsignal, S 35, dargestellt als Punkt C auf der Kurve 38', ist das Messsignal S 35 mit einem Signalabfall wieder auf das Grundsignal vor der Dosierung der Prüfstoffmenge 5, 6 (Figuren 1a, 1b, 1c und 1d) mittels der Steuersignal 91', 91" angelangt.

Für den Fall, dass bei einem optischen Gassensor 300 (Figur 1 a) oder einem Halbleitergassensor 303 (Figur 1d) das Gaszutrittselement 8 (Figur 1a, 1d) eine Blockade oder einen Verschluss im Gaszutritt 7 (Figur 1a, 1d) aufweist, ergibt sich ein Signalverlauf 38'". Für den Fall, dass für einen katalytischen Gassensor 301 (Figur 1b) oder für einen elektrochemischen Gassensor 302 (Figur 1c) das Gaszutrittselement 8 (Figur 1b, 1c) eine Blockade aufweist, ergibt sich ein Signalverlauf 38".

Das zweite Diagramm 22 zeigt den Verlauf des Steuersignals 91 über dem Zeitverlauf t 400. Dieser Zeitverlauf t 400 des zweiten Diagramms 22 ist synchron zum Zeitverlauf t 400 des ersten Diagramms 21 dargestellt. Beginnend von einem Zeitpunkt t₀ 410 wird zu einem Zeitpunkt t₁ 401 ein Steuersignal 91' aktiviert, welches die Dosierung der Prüfstoffmenge 5 (Figuren 1a, 1b, 1c und 1d) bewirkt. Zum Zeitpunkt t₂ 402 erfolgt die Deaktivierung der Dosierung der Prüfstoffmenge 5 (Figuren 1a, 1b, 1c und 1d) mittels des Steuersignals 91".

Das dritte Diagramm 23 dieser Figur 2 zeigt ausschnittsweise zwei hervorgehobene Zeiträume 408 und 409 im Zeitverlauf t 400. Diese beiden Zeiträume 408, 409 sind für die Überprüfung der Anordnung zur Gasmessung1, 1', 1", 1'" (Figuren 1a, 1 b, 1 c und 1 d) zur Einordnung im Zeitverlauf t 400 relevant. Die Prozesszeitdauer 408 ist die Zeitdauer, welche sich bei einem ungehinderten Ein- und Ausströmen von Gas aus der Messumgebung 2 (Figuren 1a, 1 b, 1 c und 1 d) durch das Gaszutrittselement 8 (Figuren 1a, 1 b, 1 c und 1 d) hin zum Gassensor 30, 300, 301, 302, 303 (Figuren 1a, 1 b, 1 c und 1 d) gemäß den Kurvenverlauf 38' aus dem ersten Diagramm 21 ergibt. Die Prozesszeitdauer 408 ist demnach eine Zeitdauer für Ein- und Ausströmung, beginnend mit dem Zeitpunkt t_{1'}, 401', bei welchem nach der Dosierung die Umwandlung, bzw. Verdunstung oder Verdampfung von der flüssigen Prüfstoffmenge 5 in die gasförmige Prüfstoffmenge 6 (Figuren 1a, 1b, 1c und 1d) an der sensorisch-messtechnischen Anordnung (Figuren 1a, 1b, 1c und 1d) in Wirkung tritt, bis zur Ausströmung der gasförmigen Prüfstoffmenge 6 (Figuren 1a, 1b, 1c, 1d und 3) an die Messumgebung 2 (Figuren 1a, 1b, 1c, 1d und 3) durch das Gaszutrittselement 8 (Figuren 1a, 1b, 1c, 1d und 3). Effekte durch Wind oder Druckschwankungen in der Messumgebung 2 (Figuren 1a, 1b, 1c, 1d und 3) sind dabei im ersten Ansatz bei der Wahl einer geeigneten Prozesszeitdauer vernachlässigt. Der Zeitpunkt t₃ 403 ergibt sich bezogen auf den Zeitpunkt 401' t_{1'} als eine Zeitdauer der Sensorreaktion nach Verdampfung der flüssig eindosierten Prüfstoffmenge 5 (Figuren 1a, 1b, 1c und 1d) gemäß dem Kurvenverlauf 38 als Signalanstieg aus dem ersten Diagramm 21 bis zur Verdünnung des in der Gassensorik 30 (Figuren 1a, 1b, 1c und 1d) enthaltenen Gasgemisches zu einem Wert von 10% der maximalen Amplitude des Messsignals S 35 gemäß dem Kurvenverlauf 38' als Signalabfall bei ungehinderten Ausströmen von Gas in die Messumgebung 2 (Figuren 1a, 1b, 1c und 1d) aus dem ersten Diagramm 21. Im ersten Diagramm 21 ist dieser Wert mit 10% der maximalen Amplitude des Messsignals S 35 mit dem Punkt B bezeichnet. Die Prozesszeitdauer 408 entspricht also der Zeitdauer, in welcher eine als flüssige Prüfstoffmenge 5 dosierte und sodann nach Umwandlung gasförmige Prüfstoffmenge 6 (Figuren 1a, 1b, 1c und 1d) an der sensorisch-messtechnischen Anordnung wirksam ist bzw. nicht mehr wirksam ist.

Die Zeitdauer 409 beginnt mit dem Ende der zuvor beschriebenen Prozesszeitdauer 408. In den Diagrammen 21, 23 ist dargestellt, dass die Dauer der Einströmung 7 (Figuren 1a, 1b, 1c, 1d) von Gas aus der Messumgebung 2 (Figuren 1a, 1b, 1c, 1d) und die darauf folgende Dauer der Ausströmung von Gas zurück in die Messumgebung 2 (Figuren 1a, 1b, 1c, 1d) zusammen als Prozesszeitdauer 408 einer Summe aus Signal- Anstiegsphase und Signal-Abfallphase entspricht. Daraus resultiert in diesem Beispiel, dass die Unterschreitung des 10%-Amplituden-Wertes des Messsignals S 35 (Punkt B) im ersten Diagramm 21 am Ende der Prozesszeitdauer 408 und innerhalb des Erwartungsfensters 409 gelegen ist, was eine Situation beschreibt, in welcher ein ungehinderter Zutritt mit Ein- und Ausströmung zum Gaszutrittselement 8 (Figuren 1a, 1b, 1c und 1d) gegeben ist.

Eine Alternative zur Festlegung des Beginns der Zeitdauer 409 ist beispielsweise dadurch möglich, dass eine zweifache Ansprechzeit als Entsprechung zur Prozesszeitdauer 408 angesetzt wird. Eine weitere Alternative ist ermöglicht, wenn experimentell oder mittels Berechnung die jeweiligen Diffusionszeiten für Ein- und Ausströmung sowie deren Summe auf Basis der in der Praxis ausgeführten Ausgestaltung des Gaszutrittselements 8 (Figuren 1a, 1b, 1c und 1d) in der Anordnung zur Gasmessung 1, 1', 1", 1'" (Figuren 1a, 1b, 1c und 1d) und die jeweils zugehörigen Materialeigenschaften (Anzahl der Gaszutrittselemente, Porengröße, Fläche, Durchmesser) ermittelt werden und entsprechend als Prozesszeitdauer 408 hinterlegt wird.

Im ersten Diagramm 21 sind die Punkte B und C zu Zeitpunkten t_{B} 403" und tc 404" gezeichnet, wobei sie rein zufällig auf den Zeitpunkten t₃ 403, sowie t₄ 404 gelegen sind. Eine Unterschreitung des 10%-Amplitudenwertes kann aber zu einem beliebigen Zeitpunkt im Erwartungsfenster 409 gegeben sein. Wenn dieser Punkt B mit 10% der Amplitude des Messsignals S 35 im Erwartungsfenster 409 zwischen dem Zeitpunkten t₃ und t₄ gelegen ist, so kann von der Kontrolleinheit 3 (Figuren 1a, 1b, 1c und 1d) derart ausgewertet und gefolgert werden, dass die Überprüfung mit einem Ergebnis durchgeführt worden ist, dass das Gaszutrittselement 8 (Figuren 1a, 1b, 1c und 1d) ohne Blockaden oder Verschmutzungen ausreichend frei durchgängig ist.

Im ersten Diagramm 21 zeigt der Messsignalverlauf 38" einen Verlauf, welcher einer sensorisch-messtechnischen Anordnung mit einem katalytischen Gassensor 301 oder einem elektrochemischen Gassensor 302 zugehörig ist, ein abnehmendes Messsignal S 35, das sowohl ein katalytischer Gassensor 301 wie auch ein elektrochemischer Gassensor 302 einen Verbrauch an Messgas aufweisen. Dadurch sinkt das Messsignal S 35 nach Eindosierung auch dann ab, wenn kein Gas aus dem Gassensor 30 (Figuren 1a, 1b, 1c und 1d) in die Messumgebung 2 (Figuren 1a, 1b, 1c und 1d) entweichen kann, bzw. wieder ausströmen kann. Der Verlauf nach 38" zeigt somit einen Fall auf, bei dem ein Gaszutrittselement 8 (Figuren 1a, 1b, 1c und 1d) eines katalytischen Gassensors 301 oder eines elektrochemischen Gassensors 302 zur Messumgebung 2 (Figuren 1a, 1b, 1c und 1d) hin verschlossen oder im Gasaustausch gehindert ist.

Die Figur 3 zeigt einen Ablaufplan 1000 für eine Überprüfung einer Anordnung zur Gasmessung 1, 1', 1", 1'" (Figuren 1a, 1b, 1c und 1d) mit einem Start 100 und einem Ende 106 (Stopp). Der Ablauf 1000 steuert die Reihenfolge von fünf Betriebszuständen 101, 102, 103, 104, 105 vom Start 100 bis zum Ende 106. Die Kontrolleinheit 3 bedient sich dabei zur Steuerung des Ablaufs 1000 Funktionalitäten zur Zeitmessung, welche in dieser Figur 3 als den Betriebszuständen 102, 102', 103 und 104 zugewiesene Zeitgeber 44 dargestellt sind. Die Kontrolleinheit 3 wirkt über Steuersignale, Steuerleitungen 91, 91', 91" sowie Kontrollleitungen 33 auf die Betriebszustände 101, 102, 103, 104, 105, wie auch auf die Prüfgasquelle 9 ein. Der Ablauf der Zeiten, welche mittels der Zeitgeber 44 durch die Kontrolleinheit 3 gesteuert wird, wird durch ein Synchronisationssignal 34 symbolisiert.

Im ersten Betriebszustand 101 (Messbetrieb) wird in einem ersten Schritt 101 der normale Messbetrieb mit der Gassensorik 30 durchgeführt. Dabei erfasste Messsignale werden ausgewertet und dargestellt. Die dazu notwendigen Verbindungen und Elemente sind in dieser Figur 3 nicht mit dargestellt. Im Messbetrieb 101 gelangt aus einer Messumgebung 2 ein Gaszutritt 7 über ein Gaszutrittselement 8 in einen Gassensor 30 und in dem Gassensor 30 zu einer sensorisch-messtechnischen Anordnung.

In einem zweiten Betriebszustand 102 (Prüfgasgabe) wird von der Kontrolleinheit 3 mittels einer Kontrollleitung 33 die Prüfgasquelle 9 aktiviert, um eine Prüfstoffmenge 5, 6 zu dem Gassensor 30 zu dosieren. Dabei wird von der Kontrolleinheit 3 ein Zeitgeber 44 mittels eines Steuersignals 91' aktiviert und damit ein Zeitpunkt t₁ 401 markiert. Der Zeitpunkt t₁ wird dabei in einem der Kontrolleinheit 3 befindlichen Datenspeicher 32 abgelegt, so dass er für den fünften Betriebszustand (Fallunterscheidung) 105 zur Verfügung steht. Die für den Übergang der flüssig dosierten Prüfstoffmenge 5 in eine gasförmige Prüfstoffmenge 6 notwendige Verdunstungszeit, wie in Figur 1a beschrieben, ist als ein Zeitpunkt t_{1'} 401' ebenfalls dort hinterlegt. An den zweiten Betriebszustand 102 schließt sich ein weiterer Schritt 102' als Teil des zweiten Betriebszustandes 102 an, in welchem von der Kontrolleinheit 3 ein weiteres Steuersignal 91" an die Prüfgasquelle 9 gegeben wird, um die Dosierung der Prüfstoffmenge 5 zu einem Zeitpunkt t₂ 402 zu beenden. In einer Ausgestaltung der Prüfgasquelle 9 als ein Piezo-Dosierelement kann vorgesehen sein, dass der zweite Betriebszustand 102' mit der Deaktivierung der Prüfgasquelle 9 entfallen kann, da für die Dosierungsansteuerung des Piezo-Dosierelements kein Deaktivierungssignal 91" im zweiten Betriebszustand 102 erforderlich ist. Als eine weitere Ausgestaltung der Dosierung mit einem Piezo-Dosierelement kann vorgesehen sein, eine Dosierung der Prüfstoffmenge durch eine Sequenz von Tropfen einzustellen. Durch eine Vorgabe einer vorbestimmten Anzahl von Impulsen auf das Piezo-Dosierelement wird eine mit der Anzahl von Impulsen korrespondierende Anzahl von Tropfen als Prüfstoffmenge dosiert.

In einem dritten Betriebszustand 103 (Messsignalerfassung) wird von der Gassensorik 30 ein Messsignal S 35, wie auch ein Messsignalverlauf 38 auf Basis der eindosierten Prüfstoffmenge 5, 6 erfasst. Das Messsignal S 35, wie auch der Messsignalverlauf 38 werden mittels einer Messsignalbereitstellung 37 und einer Signalweiterleitung 36 dem Ablauf 1000, und damit der Kontrolleinheit 3 zur Verfügung gestellt. Zu einem Zeitpunkt t₃ 403, welcher durch den Zeitgeber 44 im dritten Betriebszustand 103 vorgegeben wird, wird das erfasste Messsignal S 35 mit einem ersten Messsignalschwellwert 350 verglichen.

In einem folgenden vierten Betriebszustand (Messsignalerfassung) 104 erfolgt eine weitere Überprüfung des Messsignals S 35 zu einem, zeitlich nach dem Zeitpunkt t₃ 403 folgenden vorgegebenen Zeitpunkt t₄ 404, in ähnlicher Weise wie im dritten Betriebszustand 103 beschrieben, mit einem Vergleich des erfassten Messsignal S 35 mit einem zweiten Messsignalschwellwert 351.

Die Werte des ersten Messsignalschwellwertes 350 und des zweiten Messsignalschwellwertes 351 werden aus einem zuvor ermittelten typischen Signalverhalten der sensorisch-messtechnischen Anordnung bei Dosierung der Prüfstoffmenge 5, 6 mit der Prüfgasquelle 9 mit ungehinderter Abströmung in die Messumgebung 2 durch das Gaszutrittselement 8 abgeleitet. Dabei gehen vorzugsweise das bereits zu Figur 2 erwähnte Ansprechverhalten (Anstiegszeit, z. B. t₁₀₋₉₀ - Anstiegszeit, Abfallzeit, z.B. t₉₀₋₁₀ - Abfallzeit), der Verlauf der Signalamplitude 35, 38 (Nullsignal, maximale Signalamplitude) bei einer ungehinderten Zuströmung 7 durch das Gaszutrittselement 8 zu der messtechnischen Anordnung und ungehinderten Abströmung in die Messumgebung 2 durch das Gaszutrittselement 8 mit ein. Dabei wird für den zweiten Messsignalschwellwert 351 ein gegenüber dem Wert des ersten Messsignalschwellwertes 351 Wert mit einer geringeren Signalamplitude gewählt, da sich bei einer ungehinderten Abströmung der dosierten Prüfstoffmenge 5, 6 aus dem Gassensor 30 durch das Gaszutrittselement 8 in die Messumgebung 2 im Verlauf des Messsignals 38 eine Konstellation mit einem Signalabfall 38', 38" (Figur 2) während des Erwartungsfensters 409 (Figur 2) ergibt.

Im fünften Betriebszustand (Fallunterscheidung) 105 werden die Ergebnisse der Vergleiche des Messsignals 35 mit den Messsignalschwellwerten 350, 351 dahingehend ausgewertet, ob das Messsignal 35 im Verlauf 38 unterhalb eines der Schwellwerte 350, 351 liegt. Sofern der Vergleich das Ergebnis bringt, dass das Messsignal 35 die Schwellwerte 350, 351 unterschritten hat, so wird in einen ersten Zustand der Gassensorik 200 verzweigt, aus dem heraus der Messbetrieb 101 fortgesetzt wird. In diesem Fall ist die Überprüfung des Gassensors 30 mittels der dosierten Prüfstoffmenge 5, 6 mit dem positiven Ergebnis beendet, dass das Gaszutrittselement 8 eine ungehinderte Einströmung 7 ermöglicht, also, dass das Gaszutrittselement 8 frei von Blockaden ist. Für den Fall, dass das Ergebnis der Überprüfung im fünften Betriebszustand 105 dahingehend ist, dass keiner der Schwellwerte 350, 351 im Verlauf 38 in einem durch die Zeitpunkte t₄ und t₃ gebildeten Erwartungsfenster 409 (Figur 2) unterschritten hat, so hat die Überprüfung der Gassensorik 30 das Ergebnis, dass das Gaszutrittselement 8 für den Gaszutritt 7 mutmaßlich blockiert ist. In diesem Fall wird in einen zweiten Zustand 800 der Gassensorik 30 verzweigt, in welchem über eine Ausgabeeinheit 80 das Ergebnis der Überprüfung bereitgestellt wird und anschließend der Ablauf 1000 beendet 106 (Stopp) wird.

### BEZUGSZEICHENLISTE

- 1, 1', 1", 1'": Anordnung zur Gasmessung, Gasmessvorrichtung
- 2: Messumgebung
- 3: Kontrolleinheit, Elektronikeinheit
- 5: Prüfstoffmenge (Flüssig), eingespritzt
- 6: Prüfstoffmenge (gasförmig), verdampft
- 7: Gaszutritt
- 8: Gaszutrittselement
- 9: Einheit zur Prüfgasdosierung
- 21: erstes Diagramm
- 22: zweites Diagramm
- 23: drittes Diagramm
- 30: Gassensorik
- 32: Datenspeicher (RAM, ROM)
- 33: Kontrollleitung
- 35: Messsignal S, Messsignalleitung
- 36: Signalweiterleitung
- 38: Messsignalverlauf
- 37: Signalbereitstellung
- 40: akustischer Alarmgeber (Hupe)
- 44: Zeitgeber / Stoppuhr / Chronometer
- 50: optischer Alarmgeber (Lampe)
- 60: Bedien-und Darstellungseinheit (User-Interface)
- 61: Bildschirmelement
- 70: Auswertesystem
- 71: Datenbank
- 80: Ausgabeeinheit
- 81: Schnittstelle
- 91, 91', 91": Steuersignal, Steuersignalverlauf, Steuerleitung
- 92: Signal- und Datenleitung, Zustandssignal oder Alarmsignal
- 100: Start
- 101: Erster Betriebszustand (Messbetrieb) / Schritt 1
- 102, 102': Zweiter Betriebszustand (Prüfgasgabe) / Schritte 2, 2'
- 103: Dritter Betriebszustand (Messsignalerfassung) / Schritt 3
- 104: Vierter Betriebszustand (Messsignalerfassung) / Schritt 4
- 105: Fünfter Betriebszustand (Fallunterscheidung) / Schritt 5
- 106: Stopp
- 200: erster Zustand der Gassensorik
- 300: optischer Gassensor, IR-Multireflexionszelle
- 301: katalytischer Gassensor, Wärmetönungssensor,
- 302: Elektrochemischer Gassensor
- 303: Halbleiter-Gassensor
- 304: Ventil
- 305: Prüfstoffvorrat, Tank, Behältnis, Flasche
- 306: Vorratsmenge sensorisch-messtechnische Anordnung
- 350: erster Messsignal-Schwellwert (t₃, t_{B})
- 351: zweiter Messsignal-Schwellwert (t₄, t_{C})
- 400: x- Achse, Zeitverlauf t
- 401: Zeitpunkt t₁, Aktivierungszeitpunkt
- 401': Zeitpunkt t_{1'}
- 401": Zeitpunkt t_{A}
- 402: Zeitpunkt t₂, Deaktivierungszeitpunkt
- 403: Zeitpunkt t₃
- 403": Zeitpunkt t_{B}
- 404: Zeitpunkt t₄
- 404": Zeitpunkt t_{C}
- 408: Prozesszeitdauer T_{|t3-t1'|} (Einströmung + Verdunstung + Ausströmung)
- 409: Erwartungsfenster T_{|t4- t3|}
- 410: Zeitpunkt t₀
- 800: zweiter Zustand der Gassensorik
- 1000: Ablauf

## Patentansprüche

1. Verfahren zur Überprüfung mindestens eines Gaszutrittselements (8) eines Gassensors (30) oder einer Anordnung zur Gasmessung (1, 1', 1", 1'") mit mindestens einem Gassensor (30),
wobei eine Kontrolleinheit (3) von einem Startzeitpunkt (100, 410), beginnend mit einem ersten Betriebszustand (101) mit einem kontinuierlichen Messbetrieb eine Abfolge von Betriebszuständen (101, 102, 103, 104, 105, 106) derart steuert, dass
- die Kontrolleinheit (3) in einem zweiten Betriebszustand (102) eine Dosierung einer Prüfstoffmenge (5, 6) mittels einer stromabwärts des Gaszutrittselements (8) und stromaufwärts der sensorisch-messtechnischen Anordnung angeordneten Einheit zur Prüfgasdosierung (9) zu einer in dem Gassensor (30) angeordneten sensorisch-messtechnischen Anordnung bewirkt,
- die Kontrolleinheit (3) in einem dritten Betriebszustand (103) einen Beginn eines Erwartungszeitfensters (409) initiiert (403), die Kontrolleinheit (3) mit dem Beginn des Erwartungszeitfensters (409) mindestens ein Messsignal (35) oder eine Vielzahl von Messsignalen (38) des Gassensors (30) erfasst und die Kontrolleinheit (3) das mindestens eine erfasste Messsignal (35) oder eines der Vielzahl von Messsignalen (38) mit einem ersten Messsignal-Schwellwert (350) vergleicht,
- die Kontrolleinheit (3) in einem vierten Betriebszustand (103) mindestens ein weiteres Messsignal (35) oder eine weitere Vielzahl von Messsignalen (38) des Gassensors (30) erfasst und die Kontrolleinheit (3) das mindestens eine weitere erfasste Messsignal (35) oder eines der weiteren Vielzahl von Messsignalen (38) mit einem zweiten Messsignal-Schwellwert (351) vergleicht und die Kontrolleinheit (3) ein Ende des Erwartungszeitfensters 409 initiiert,
- die Kontrolleinheit (3) in einem fünften Betriebszustand (105) auf Basis des Vergleichs des Messsignals (35) mit dem ersten Messsignal-Schwellwert (350) und/oder mit dem zweiten Messsignal-Schwellwert (351) bestimmt, ob das Gaszutrittselement (8) zu einer Zuführung (7) von Luft, Gas oder Gasgemisch aus einer Messumgebung (2) funktionsbereit ist
und die Kontrolleinheit (3) auf Basis des Vergleichs des Messsignals (35) mit dem ersten Messsignal-Schwellwert (350) und/oder mit dem zweiten Messsignal-Schwellwert (351) ein Maß für eine Funktionsbereitschaft des Gassensors (30) bestimmt.

2. Verfahren nach Anspruch 1, wobei in dem zweiten Betriebszustand (102) von der Kontrolleinheit (3) die Dosierung der flüssigen Prüfstoffmenge (5) von der Einheit zur Prüfgasdosierung (9) zu der sensorisch-messtechnischen Anordnung in dem Gassensor (30) derart bewirkt wird, dass die Kontrolleinheit (3) im Zeitverlauf t (400) zu einem ersten Zeitpunkt (Aktivierungszeitpunkt) t₁ (401) die Einheit zur Prüfgasdosierung (9) aktiviert (91').

3. Verfahren nach Anspruch 1, wobei in dem zweiten Betriebszustand (102) von der Kontrolleinheit (3) die Dosierung der flüssigen Prüfstoffmenge (5) von der Einheit zur Prüfgasdosierung (9) zu der sensorisch-messtechnischen Anordnung in dem Gassensor (30) derart bewirkt wird, dass die Kontrolleinheit (3) im Zeitverlauf t (400) zu dem ersten Zeitpunkt (Aktivierungszeitpunkt) t₁ (401) die Einheit zur Prüfgasdosierung (9, 304) zur Dosierung der flüssigen Prüfstoffmenge (5) aktiviert (91') und,
dass die Kontrolleinheit (3) im Zeitverlauf t (400) zu einem zweiten Zeitpunkt (Deaktivierungszeitpunkt) t₂ (402), welcher von dem ersten Zeitpunkt (Aktivierungszeitpunkt) t₁ (401) zeitlich nachfolgend beabstandet ist, die Dosierung der flüssigen Prüfstoffmenge (5) durch die Einheit zur Prüfgasdosierung (9, 304) deaktiviert (91").

4. Verfahren nach Anspruch 3, wobei von der Kontrolleinheit (3) eine Größe und/oder ein Volumen des Gassensors (30) für den zweiten Zeitpunkt (Deaktivierungszeitpunkt) t₂ (402) im Zeitverlauf t (400) berücksichtigt wird.

5. Verfahren nach einem der vorhergehenden Ansprüche, wobei von der Kontrolleinheit (3) eine Größe und/oder ein Volumen des Gassensors (30) bei der Dosierung der Menge der flüssigen Prüfstoffmenge (5) mittels der Einheit zur Prüfgasdosierung (9) berücksichtigt wird.

6. Verfahren nach einem der vorhergehenden Ansprüche, wobei in dem dritten Betriebszustand (103) von der Kontrolleinheit (3) der dritte Zeitpunkt t₃ (403) von dem ersten Zeitpunkt (Aktivierungszeitpunkt) t₁ (401) im Zeitverlauf t (400),
welcher für die Dosierung der flüssigen Prüfstoffmenge (5) zu der sensorisch-messtechnischen Anordnung repräsentativ ist oder von einem,
von diesem ersten Zeitpunkt (Aktivierungszeitpunkt) (401) abgeleiteten Zeitpunkt t1' (401'), welcher für eine zeitlich nach der Dosierung erfolgte Umwandlung der flüssigen Prüfstoffmenge (5) zu einer gasförmigen Prüfstoffmenge (6) an der sensorisch-messtechnischen Anordnung repräsentativ ist,
derart ermittelt oder vorgegeben wird, dass
der dritte Zeitpunkt t₃ (403) für eine Prozesszeitdauer (408) von dem Zeitpunkt t₁ (401) oder dem Zeitpunkt t_{1'} (401') derartig zeitlich beabstandet ist, welcher mit einer ungehinderten Zuströmung (7) aus der Messumgebung (2) durch das Gaszutrittselement (8) zu der sensorisch-messtechnischen Anordnung in dem Gassensor (30) und einer anschließenden ungehinderten Ausströmung aus dem Gassensor (30) in die Messumgebung (2) korrespondierend ist oder der Zeitdauer von Zuströmung und Abströmung entspricht.

7. Verfahren nach Anspruch 6, wobei von der Kontrolleinheit (3) eine Größe und/oder ein Volumen des Gassensors (30), eine Anzahl, eine Dicke, eine Porengröße eine Fläche und/oder ein Durchmesser des Gaszutrittselements (8) für die zeitliche Dauer der Prozesszeitdauer (408) und/oder für den dritten Zeitpunkt t₃ (403) im Zeitverlauf t (400) berücksichtigt wird.

8. Verfahren nach einem der vorhergehenden Ansprüche, wobei von der Kontrolleinheit (3) im zweiten, dritten, vierten und fünften Betriebszustand (102, 102', 103, 104, 105) für die Zeit der Unterbrechung der kontinuierlichen Messung ein Ersatzsignal bereitgestellt wird.

9. Verfahren nach einem der vorhergehenden Ansprüche, wobei von der Kontrolleinheit (3) eine Größe und/oder ein Volumen des Gassensors (30), eine Anzahl, eine Dicke, eine Porengröße eine Fläche und/oder ein Durchmesser des Gaszutrittselements (8) für die zeitliche Dauer des Erwartungszeitfensters (409) und/oder für den vierten Zeitpunkt t₄ (404) im Zeitverlauf t (400) berücksichtigt wird.

10. Verfahren nach einem der vorhergehenden Ansprüche, wobei von der Kontrolleinheit (3) auf Basis des Vergleichs im fünften Betriebszustand (105) eine Ermittlung und/oder Bereitstellung eines Zustandssignals (92) erfolgt.

11. Verfahren nach Anspruch 10, wobei das Zustandssignal (92) von der Kontrolleinheit (3) an eine Ausgabeeinheit (80), ein zentrales Auswertesystem (70), eine Alarmzentrale oder ein mobiles Ausgabemittel bereitgestellt wird.

12. Verfahren nach Anspruch 10, wobei von der Ausgabeeinheit (80), dem zentralen Auswertesystem (70), der Alarmzentrale oder dem mobilen Ausgabemittel eine Ausgabe eines Alarmsignals (92) oder eine Ausgabe einer Meldung erfolgt.

13. Verfahren nach Anspruch 12, wobei das Alarmsignal (92) von der Kontrolleinheit (3) und/oder der Ausgabeeinheit (80) einem akustischen Alarmgeber (40) zu einer akustischen Alarmierung und/oder einem optischen Signalgeber (50) zu einer optischen oder visuell sichtbaren Alarmierung bereitgestellt wird.

14. Verfahren nach Anspruch 12, wobei die Meldung auf einem Datensichtgerät (60), Bildschirm (60) als ein Hinweis (61), insbesondere als ein Warnhinweis (61) oder eine Anweisung in Textform (61), grafischer Form (61) oder in symbolischer Form (61) sichtbar von der Kontrolleinheit (3) und/oder der Ausgabeeinheit (80) bereitgestellt wird.

15. Gasmessvorrichtung (1, 1', 1") zur Durchführung des Verfahrens nach einem der vorstehenden Verfahrensansprüche,
aufweisend mindestens einen Gassensor (30) mit mindestens einer sensorisch-messtechnischen Anordnung, eine Einheit zur Prüfgasdosierung (9), eine Kontrolleinheit (3), einen der Kontrolleinheit (3) zugeordneten Datenspeicher (32), wobei der Gassensor (30) oder die Anordnung zur Gasmessung (1, 1', 1", 1'")
- mindestens eine sensorisch-messtechnische Anordnung zu einer Erfassung einer Gaskonzentration oder einer Gaskonzentrationsänderung und
- das Gaszutrittselement (8) aufweist,
wobei das Gaszutrittselement (8) stromaufwärts der sensorisch-messtechnischen Anordnung angeordnet ist,
wobei die Einheit zur Prüfgasdosierung (9) stromabwärts des Gaszutrittselements (8) in dem Gassensor (30) oder in der Anordnung zur Gasmessung (1, 1', 1", 1'") angeordnet ist.

16. Gasmessvorrichtung (1, 1', 1") nach Anspruch 15,
wobei die Einheit zur Prüfgasdosierung (9) ein Piezo-Dosierelement (99) und ein, mit dem Piezo-Dosierelement (99) fluidisch verbundenes Reservoir (305) zu einer Lagerung einer Vorratsmenge (306) aufweist, wobei die Kontrolleinheit (3) zu einer Aktivierung (91') des Piezo-Dosierelementes (99) zu einem ersten Zeitpunkt t₁ (401) ausgebildet ist.

17. Gasmessvorrichtung (1, 1', 1") nach Anspruch 15,
wobei die Einheit zur Prüfgasdosierung (9) ein Ventil (304) und ein mit dem Ventil (304) fluidisch verbundenes Reservoir (305) zur Lagerung der Vorratsmenge (306) aufweist, wobei die Kontrolleinheit (3) zu einer Aktivierung (91') des Ventiles (304) zu einem ersten Zeitpunkt t₁ (401) und zu einer Deaktivierung (91 ") des Ventiles (304) zu einem zweiten Zeitpunkt t₂ (402) ausgebildet ist.

18. Gasmessvorrichtung (1, 1', 1", 1"') nach einem der vorstehenden Vorrichtungsansprüche, mit einer Ausgabeeinheit (80), einem optischen Alarmgeber (50) und/oder einem akustischen Alarmgeber (40), wobei der optische Alarmgeber (50) und/oder akustische Alarmgeber (40) in Zusammenwirkung mit der Kontrolleinheit (3) und/oder Ausgabeeinheit (80) zu einer Ausgabe eines Alarmsignals (92) ausgebildet und vorgesehen ist.

19. Gasmessvorrichtung (1, 1', 1", 1'") nach einem der vorstehenden Vorrichtungsansprüche, wobei die Ausgabeeinheit (80) eine Schnittstelle (81) aufweist, wobei die Schnittstelle (81) in Zusammenwirkung mit der Kontrolleinheit (3) zu einer Übermittlung des Zustandssignals an ein Auswertesystem (70) ausgebildet und vorgesehen ist.

20. Gasmessvorrichtung (1, 1', 1", 1'") nach einem der vorstehenden Vorrichtungsansprüche, wobei die mindestens eine sensorischmesstechnische Anordnung
- als eine Kombination von Elektroden und einem Elektrolyten in einem elektrochemischen Gassensor (302),
- als eine Kombination einer Strahlungsquelle und eines Detektorelements in einem Infrarotoptischen Gassensor (300),
- als eine Kombination von katalytisch aktiven
und/oder katalytisch passiven Messelemente in einem katalytischen Gassensor (301) oder einem Wärmetönungssensor oder
- als gasartspezifische und sensitive Halbleiter-Elemente in einem Halbleiter-Gassensor (303) ausgestaltet sind.

21. Gaswarnsystem (70) mit einer Gasmessvorrichtung (1, 1', 1", 1'"), aufweisend eine Gasmessvorrichtung (1, 1', 1", 1"') mit mindestens einem Gassensor (30) mit mindestens einem Gaszutrittselements (8) des Gassensors (30), wobei das Gaswarnsystem (70) eine Überprüfung des mindestens einen Gaszutrittselements (8) in folgender Weise koordiniert:
- Deaktivierung einer Ausgabe (92) von diesem Gassensor (30) oder dieser Gasmessvorrichtung (1, 1', 1", 1"'), zugehörigen Alarmierungen (92) und/oder Störungsmeldungen in dem Gaswarnsystem (70)
- Aktivierung (101) einer Dosierung einer Prüfstoffmenge (5, 6) mittels der Einheit zur Prüfgasdosierung zu einer in dem Gassensor (30) angeordneten sensorisch-messtechnischen Anordnung,
- Initialisierung einer vorbestimmten Wartezeit (100, 410), wobei die Dauer der Wartezeit (100, 410), einer Zeitdauer entspricht, in welcher die eindosierte Prüfstoffmenge (5, 6) in den Gassensor (30) durch das Gaszutrittselement (8) hinein und anschließend aus dem Gassensor (30) durch ein funktionsbereites Gaszutrittselement (8) wieder hinausgeströmt, bzw. hinausdiffundiert ist,
- Reaktivierung der Ausgabe (92) von diesem Gassensor (30) oder dieser Gasmessvorrichtung (1, 1', 1", 1'") zugehörigen Alarmierungen (92) und/oder Störungsmeldungen in dem Gaswarnsystem (70) nach Ablauf der vorbestimmten Wartezeit.
